(19) Europäisches Patentamt
European Patent Office
Office européen des brevets

(11) EP 4 190 788 A1

(12) **EUROPEAN PATENT APPLICATION**
published in accordance with Art. 153(4) EPC

(43) Date of publication:
**07.06.2023 Bulletin 2023/23**

(21) Application number: **21850262.3**

(22) Date of filing: **27.07.2021**

(51) International Patent Classification (IPC):
***C07D 495/04*** *(2006.01)* ***H01L 51/46*** *(2006.01)*

(52) Cooperative Patent Classification (CPC):
**C07D 495/04; H10K 85/00;** Y02E 10/549; Y02P 70/50

(86) International application number:
**PCT/JP2021/027782**

(87) International publication number:
**WO 2022/025074 (03.02.2022 Gazette 2022/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.07.2020 JP 2020129304**

(71) Applicant: **Hodogaya Chemical Co., Ltd.**
**Tokyo, 105-0021 (JP)**

(72) Inventors:
- **OHKURA Yuya**
  **Tokyo 105-0021 (JP)**
- **TAKAHASHI Hideaki**
  **Tokyo 105-0021 (JP)**
- **SATO Hiroshi**
  **Tokyo 105-0021 (JP)**

(74) Representative: **Müller-Boré & Partner Patentanwälte PartG mbB**
**Friedenheimer Brücke 21**
**80639 München (DE)**

(54) **COMPOUND, HOLE TRANSPORT MATERIAL FOR PHOTOELECTRIC CONVERSION ELEMENT, HOLE TRANSPORT LAYER, AND PHOTOELECTRIC CONVERSION ELEMENT AND SOLAR CELL USING SAME**

(57) A compound represented by general formula (1), where $X^1$ and $X^2$ are each independently represented by general formula (2).

[Chem. 1]

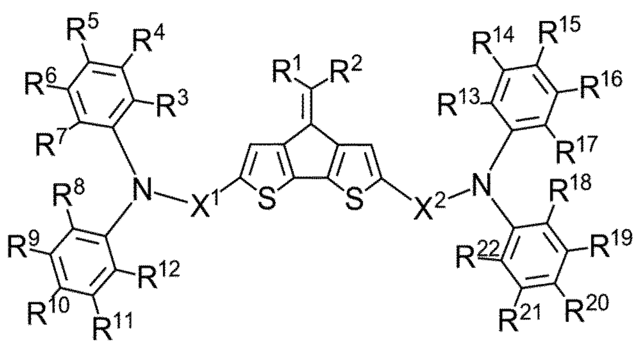

(1)

EP 4 190 788 A1

[Chem. 2]

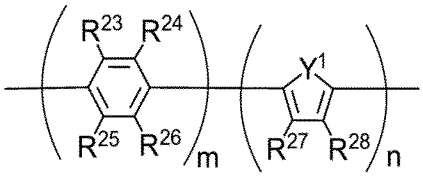

(2)

[FIG. 1]

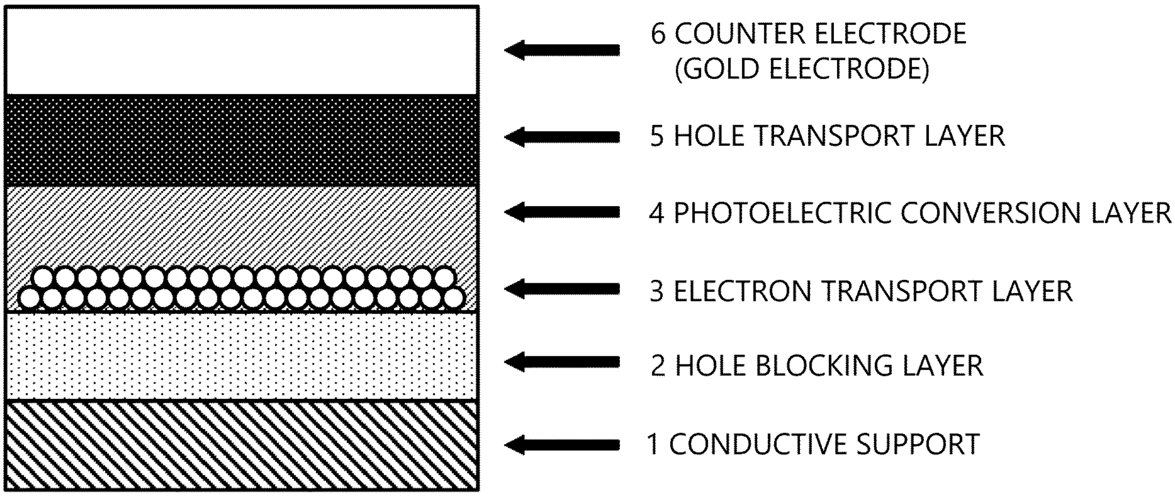
6 COUNTER ELECTRODE
(GOLD ELECTRODE)
5 HOLE TRANSPORT LAYER
4 PHOTOELECTRIC CONVERSION LAYER
3 ELECTRON TRANSPORT LAYER
2 HOLE BLOCKING LAYER
1 CONDUCTIVE SUPPORT

## Description

### TECHNICAL FIELD

The present invention relates to a compound, a hole transport material for a photoelectric conversion element, a hole transport layer, and a photoelectric conversion element and a solar cell using the same.

### BACKGROUND ART

In recent years, solar power generation has attracted attention as clean energy, and solar cells have been actively developed. Among them, development of a solar cell using a perovskite material for a photoelectric conversion layer (hereinafter, referred to as a perovskite solar cell) has attracted attention as a next-generation solar cell that can be manufactured at a low cost with a solution process (for example, Patent Literature 1 and Non-Patent Literatures 1 and 2).

In the perovskite solar cell, a hole transport material is often used in an element. The purpose of the use is to, for example, (1) improve a photoelectric conversion efficiency by increasing a function of selectively transporting holes, and (2) protect the perovskite material, which is likely to be affected by moisture and oxygen, by bonding to a perovskite photoelectric conversion layer (for example, Non-Patent Literature 3). Spiro-OMeTAD as a spirobifluorene organic compound is often used as a standard hole transport material, and there are few reports on hole transport materials that contribute more to a photoelectric conversion characteristic than this material.

In the case where the hole transport material is used for an element of a perovskite solar cell, a dopant as an additive is added to the hole transport material in a related-art method of producing the element by using an organic compound as the hole transport material. The dopant is used to reduce an electrical resistance of the hole transport material (for example, Non-Patent Literatures 3 and 4).

However, the use of the dopant as the additive may reduce durability of a photoelectric conversion element using an organic compound to shorten a life of the entire element (for example, Non-Patent Literature 3), and thus it is desired to develop a photoelectric conversion element including a hole transport layer containing no dopant or having a reduced dopant content. In addition, when the dopant is not contained or the content thereof can be reduced, an additive cost and a manufacturing process cost can be reduced.

### CITATION LIST

### PATENT LITERATURE

Patent Literature 1: WO2017/104792

### NON-PATENT LITERATURE

Non-Patent Literature 1: Journal of the American Chemical Society, 2009, Vol. 131, P. 6050-6051
Non-Patent Literature 2: Science, 2012, Vol. 388, P. 643-647
Non-Patent Literature 3: Chem. Sci., 2019, 10, P. 6748-6769
Non-Patent Literature 4: Adv. Funct. Mater., 2019, 1901296

### SUMMARY OF INVENTION

### TECHNICAL PROBLEM

An object of the present invention is to provide a novel hole transport material for a photoelectric conversion element, which can be used for a photoelectric conversion element and a solar cell to form a photoelectric conversion element and a solar cell with high durability, and a photoelectric conversion element and a solar cell using the same.

### SOLUTION TO PROBLEM

As a result of intensive studies for solving the above problem, the present inventors have found that a photoelectric conversion element and a solar cell with high durability can be obtained by using a compound having a specific structure as a hole transport material for a photoelectric conversion element. That is, the present invention is summarized

as follows.

1. A compound represented by the following general formula (1), in which $X^1$ and $X^2$ in the general formula (1) are each independently represented by the following general formula (2).

[Chem. 1]

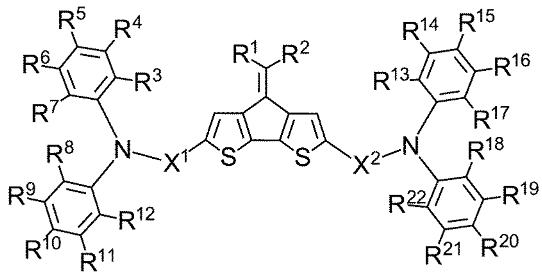

(1)

[Here, $R^1$ and $R^2$ each independently represent

a nitrile group,
a linear or branched perfluoroalkyl group having 1 to 4 carbon atoms,
a linear or branched acyl group having 1 to 18 carbon atoms which may have a substituent,
a linear or branched alkoxycarbonyl group having 1 to 18 carbon atoms which may have a substituent,
a thio group having 1 to 18 carbon atoms which may have a substituent,
an amino group having 1 to 20 carbon atoms which may have a substituent, or
a linear or branched alkylsulfonyl group having 1 to 18 carbon atoms which may have a substituent.

$R^3$ to $R^{22}$ each independently represent

a hydrogen atom, a halogen atom, a carboxy group, a trimethylsilyl group,
a linear or branched alkyl group having 1 to 18 carbon atoms which may have a substituent,
a linear or branched alkenyl group having 2 to 20 carbon atoms which may have a substituent,
a linear or branched alkynyl group having 2 to 20 carbon atoms which may have a substituent,
a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
an acyl group having 1 to 20 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms and having a substituent, or
a heterocyclic group having 5 to 36 ring atoms which may have a substituent.

$R^1$ and $R^2$ may be bonded to each other to form a ring,
$R^3$ to $R^7$, $R^8$ to $R^{12}$, $R^{13}$ to $R^{17}$, and $R^{18}$ to $R^{22}$ may be bonded to each other between adjacent groups to form a ring, and
$R^7$ and $R^8$, and $R^{17}$ and $R^{18}$ may be bonded to each other to form a ring.
However, when $R^1$ and $R^2$ are a nitrile group, and $R^5$ is a methoxy group, $R^{10}$, $R^{15}$, and $R^{20}$ are at least one of the above groups other than a methoxy group.]

[Chem. 2]

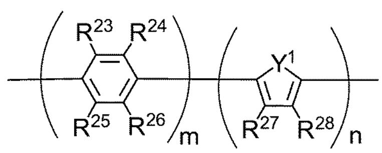

(2)

[Here, $R^{23}$ to $R^{28}$ each independently represent

a hydrogen atom,
a linear or branched alkyl group having 1 to 20 carbon atoms which may have a substituent,
a linear or branched alkenyl group having 2 to 20 carbon atoms which may have a substituent,
a linear or branched alkynyl group having 2 to 20 carbon atoms which may have a substituent,
a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms which may have a substituent, or
a heterocyclic group having 5 to 36 ring atoms which may have a substituent.

$R^{23}$ and $R^{24}$, $R^{25}$ and $R^{26}$, and $R^{27}$ and $R^{28}$ may be bonded to each other to form a ring.
$Y^1$ represents an oxygen atom, a sulfur atom, or a selenium atom, and
m and n each represent an integer of 0 to 2.
However, any one of m and n is 1 or 2.]

2. The compound according to the above 1, in which in the general formula (1), $R^1$ and $R^2$ are each independently

a nitrile group,
a linear or branched acyl group having 1 to 18 carbon atoms which may have a substituent, or
a linear or branched alkoxycarbonyl group having 1 to 18 carbon atoms which may have a substituent.

3. The compound according to the above 1 or 2, in which m in the general formula (2) is 1.
4. A hole transport material for a photoelectric conversion element, the material containing the compound according to any one of the above 1 to 3.
5. A hole transport layer, containing: a composition containing the hole transport material for a photoelectric conversion element according to the above 4, and a dopant as an additive in an amount of 0 equivalents to 0.5 equivalents with respect to 1 equivalent of the hole transport material for a photoelectric conversion element.
6. A photoelectric conversion element, containing: the hole transport material for a photoelectric conversion element according to the above 5.
7. A solar cell, including: the photoelectric conversion element according to the above 6.

## ADVANTAGEOUS EFFECTS OF INVENTION

According to the compound in the present invention and the hole transport material for a photoelectric conversion element containing the compound, a photoelectric conversion element and a solar cell with high durability can be produced.

## BRIEF DESCRIPTION OF DRAWINGS

FIG. 1 is a first schematic cross-sectional view illustrating a configuration of a photoelectric conversion element in Examples and Comparative Example of the present invention.
FIG. 2 is a second schematic cross-sectional view illustrating a configuration of a photoelectric conversion element in Examples and Comparative Example of the present invention.

## DESCRIPTION OF EMBODIMENTS

Hereinafter, an embodiment of the present invention is described in detail with reference to the drawings. A hole transport material for a photoelectric conversion element according to the present invention is used in a photoelectric conversion element and a solar cell.

### (Photoelectric Conversion Element)

In a typical example, as illustrated in a first schematic cross-sectional view of FIG. 1, a photoelectric conversion element according to the present invention includes a conductive support 1, a hole blocking layer 2, an electron transport

layer 3, a photoelectric conversion layer 4, a hole transport layer 5, and a counter electrode 6. In another typical example, as illustrated in a second schematic cross-sectional view of FIG. 2, the photoelectric conversion element according to the present invention includes a conductive support 7, an electron transport layer 8, a photoelectric conversion layer 9, a hole transport layer 10, and a counter electrode 11.

Hereinafter, a compound represented by a general formula (1), which is the hole transport material for a photoelectric conversion element according to the present invention, will be described in detail, but the present invention is not limited thereto.

In the compound represented by the general formula (1), $R^1$ and $R^2$ each independently represent

a nitrile group,
a linear or branched perfluoroalkyl group having 1 to 4 carbon atoms,
a linear or branched acyl group having 1 to 18 carbon atoms which may have a substituent,
a linear or branched alkoxycarbonyl group having 1 to 18 carbon atoms which may have a substituent,
a thio group having 1 to 18 carbon atoms which may have a substituent,
an amino group having 1 to 20 carbon atoms which may have a substituent, or
a linear or branched alkylsulfonyl group having 1 to 18 carbon atoms which may have a substituent, and $R^1$ and $R^2$ are preferably electron withdrawing.

In the present invention, examples of the "halogen atom" include fluorine, chlorine, bromine, and iodine.

Specific examples of the "linear or branched perfluoroalkyl group having 1 to 4 carbon atoms" represented by $R^1$ and $R^2$ in the general formula (1) include a methyl group, an ethyl group, an n-propyl group, and an n-butyl group of which hydrogen atoms are completely substituted with fluorine atoms (perfluorinated) in a linear or branched alkyl group having 1 to 4 carbon atoms.

Specific examples of the "linear or branched acyl group having 1 to 20 carbon atoms" in the "linear or branched acyl group having 1 to 20 carbon atoms which may have a substituent" represented by $R^1$ and $R^2$ in the general formula (1) include an acetyl group, a propionyl group, a butyryl group, an isobutyryl group, a valeryl group, an isovaleryl group, a benzoylacetyl group, and a benzoyl group, and include a group in which hydrogen atoms are completely substituted with fluorine atoms (perfluorinated) when an alkyl chain is contained. In addition, a group bonded to an amino group (-CO-N<) may be included.

Specific examples of the "linear or branched alkoxycarbonyl group having 1 to 18 carbon atoms" in the "linear or branched alkoxycarbonyl group having 1 to 18 carbon atoms which may have a substituent" represented by $R^1$ and $R^2$ in the general formula (1) include a methoxycarbonyl group and an ethoxycarbonyl group, and include a group in which hydrogen atoms are completely substituted with fluorine atoms (perfluorinated) when an alkyl chain is contained.

Specific examples of the "thio group having 1 to 18 carbon atoms" in the "thio group having 1 to 18 carbon atoms which may have a substituent" represented by $R^1$ and $R^2$ in the general formula (1) include a methylthio group, an ethylthio group, a propylthio group, a phenylthio group, and a biphenylthio group.

Specific examples of the "amino group having 1 to 20 carbon atoms" in the "amino group having 1 to 20 carbon atoms which may have a substituent" represented by $R^1$ and $R^2$ in the general formula (1) include an ethylamino group, an acetylamino group, and a phenylamino group as a monosubstituted amino group, and a diethylamino group, a diphenylamino group, and an acetylphenylamino group as a disubstituted amino group.

The "linear or branched alkylsulfonyl group having 1 to 18 carbon atoms" in the "linear or branched alkylsulfonyl group having 1 to 18 carbon atoms which may have a substituent" represented by $R^1$ and $R^2$ in the general formula (1) is specifically a group represented by $-SO_2A^1$ ($A^1$ represents a linear or branched alkyl group having 1 to 18 carbon atoms among the above-described linear or branched alkyl groups having 1 to 20 carbon atoms which may have a substituent). Examples of $A^1$ include a methyl group, an ethyl group, an n-propyl group, and an n-butyl group described as examples of the above-described linear or branched alkyl group having 1 to 20 carbon atoms which may have a substituent.

Specific examples of the "substituent" in the "acyl group having 1 to 20 carbon atoms which may have a substituent", the "linear or branched alkoxycarbonyl group having 1 to 18 carbon atoms which may have a substituent", the "thio group having 1 to 18 carbon atoms which may have a substituent", the "amino group having 1 to 20 carbon atoms which may have a substituent", or the "linear or branched alkylsulfonyl group having 1 to 18 carbon atoms which may have a substituent" represented by $R^1$ and $R^2$ in the general formula (1) include

a halogen atom such as a fluorine atom, a chlorine atom, a bromine atom, and an iodine atom; a cyano group; a hydroxy group; a nitro group; a nitroso group; a carboxy group; a phosphate group; a thioxo group (>C=S);
a carboxylic acid ester group such as a methyl ester group and an ethyl ester group;
a linear or branched alkyl group having 1 to 18 carbon atoms, such as a methyl group, an ethyl group, an n-propyl group, an isopropyl group, an n-butyl group, an isobutyl group, an s-butyl group, a t-butyl group, an n-pentyl group,

an isopentyl group, an n-hexyl group, a 2-ethylhexyl group, a heptyl group, an octyl group, an isooctyl group, a nonyl group, and a decyl group;

a linear or branched alkenyl group having 2 to 20 carbon atoms, such as an ethenyl group (vinyl group), a 1-propenyl group, a 2-propenyl group (allyl group), a 1-butenyl group, a 2-butenyl group, a 1-pentenyl group, a 1-hexenyl group, a 2-methyl-1-propenyl group, a 2-methyl-2-propenyl group, and a 1-ethylethenyl group;

a linear or branched alkoxy group having 1 to 18 carbon atoms, such as a methoxy group, an ethoxy group, a propoxy group, a t-butoxy group, a pentyloxy group, and a hexyloxy group;

an aromatic hydrocarbon group having 6 to 30 carbon atoms, such as a phenyl group, a naphthyl group, an anthryl group, a phenanthryl group, and a pyrenyl group;

a heterocyclic group having 5 to 30 ring atoms, such as a pyridyl group, a pyrimidinyl group, a triazinyl group, a thienyl group, a furyl group (furanyl group), a pyrrolyl group, an imidazolyl group, a pyrazolyl group, a triazolyl group, a quinolyl group, an isoquinolyl group, a naphthylidinyl group, an acridinyl group, a phenanthrolinyl group, a benzofuranyl group, a benzothienyl group, an oxazolyl group, an indolyl group, a carbazolyl group, a benzoxazolyl group, a thiazolyl group, a benzothiazolyl group, a quinoxalinyl group, a benzimidazolyl group, a pyrazolyl group, a dibenzofuranyl group, a dibenzothienyl group, and a carbonyl group;

an amino group having 0 to 18 carbon atoms, which includes an unsubstituted amino group ($-NH_2$), a monosubstituted amino group such as an ethylamino group, an acetylamino group, and a phenylamino group, and a disubstituted amino group such as a diethylamino group, a diphenylamino group, and an acetylphenylamino group; and

a thio group having 0 to 18 carbon atoms, such as an unsubstituted thio group (thiol group: -SH), a methylthio group, an ethylthio group, a propylthio group, a phenylthio group, and a biphenylthio group.

Only one of these "substituents" may be included, or a plurality of "substituents" may be included, and when a plurality of "substituents" are included, the "substituents" may be the same as or different from each other. These "substituents" may further include the substituents exemplified above.

In the general formula (1), $R^1$ and $R^2$ may be bonded to each other to form a ring, or may be bonded to each other by a single bond, a bond via an oxygen atom, a sulfur atom, or a selenium atom, or a bond via a nitrogen atom to form a ring, and in this case, it is preferable to form a ring via a single bond. More preferred are acidic heterocycles of barbiturates, thiobarbiturates, rhodanines, thiohydantoins, indanediones, and the like.

In the general formula (1), $R^3$ to $R^{22}$ each independently represent

a hydrogen atom, a halogen atom, a carboxy group, a trimethylsilyl group,
a linear or branched alkyl group having 1 to 18 carbon atoms which may have a substituent,
a linear or branched alkenyl group having 2 to 20 carbon atoms which may have a substituent,
a linear or branched alkynyl group having 2 to 20 carbon atoms which may have a substituent,
a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
an acyl group having 1 to 20 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms and having a substituent, or
a heterocyclic group having 5 to 36 ring atoms which may have a substituent.

In the present invention, examples of the "halogen atom" include fluorine, chlorine, bromine, and iodine.

Specific examples of the "linear or branched alkyl group having 1 to 20 carbon atoms" in the "linear or branched alkyl group having 1 to 20 carbon atoms which may have a substituent" represented by $R^3$ to $R^{22}$ in the general formula (1) include a methyl group, an ethyl group, an n-propyl group, an isopropyl group, an n-butyl group, an isobutyl group, an s-butyl group, a t-butyl group, an n-pentyl group, an isopentyl group, an n-hexyl group, a 2-ethylhexyl group, a heptyl group, an octyl group, an isooctyl group, a nonyl group, and a decyl group.

Specific examples of the "linear or branched alkenyl group having 2 to 20 carbon atoms" in the "linear or branched alkenyl group having 2 to 20 carbon atoms which may have a substituent" represented by $R^3$ to $R^{22}$ in the general formula (1) include an ethenyl group (vinyl group), a 1-propenyl group, a 2-propenyl group (allyl group), a 1-methylethenyl group, a 1-butenyl group, a 2-butenyl group, a 1-pentenyl group, a 1-hexenyl group, a 2-methyl-1-propenyl group, a 2-methyl-2-propenyl group, a 1-ethylethenyl group, or a linear or branched alkenyl group having 2 to 20 carbon atoms to which a plurality of these alkenyl groups are bonded.

Specific examples of the "linear or branched alkynyl group having 2 to 20 carbon atoms" in the "linear or branched alkynyl group having 2 to 20 carbon atoms which may have a substituent" represented by $R^3$ to $R^{22}$ in the general formula (1) include an ethynyl group, a 1-propynyl group, a 2-propynyl group, a 1-butynyl group, a 2-butynyl

group, a 1-methyl-2-propynyl group, a 1-pentynyl group, a 2-pentynyl group, a 1-methyl-n-butynyl group, a 2-methyl-n-butynyl group, a 3-methyl-n-butynyl group, and a 1-hexynyl group.

Specific examples of the "cycloalkyl group having 3 to 10 carbon atoms" in the "cycloalkyl group having 3 to 10 carbon atoms which may have a substituent" represented by $R^3$ to $R^{22}$ in the general formula (1) include a cyclopropyl group, a cyclobutyl group, a cyclopentyl group, a cyclohexyl group, a cycloheptyl group, a cyclooctyl group, a cyclodecyl group, a cyclododecyl group, a 4-methylcyclohexyl group, and a 4-ethylcyclohexyl group.

Specific examples of the "linear or branched alkoxy group having 1 to 20 carbon atoms" in the "linear or branched alkoxy group having 1 to 20 carbon atoms which may have a substituent" represented by $R^3$ to $R^{22}$ in the general formula (1) include a methoxy group, an ethoxy group, a propoxy group, an n-butoxy group, an n-pentyloxy group, an n-hexyloxy group, a heptyloxy group, an octyloxy group, a nonyloxy group, a decyloxy group, an isopropoxy group, an isobutoxy group, an s-butoxy group, a t-butoxy group, an isooctyloxy group, a t-octyloxy group, a phenoxy group, a tolyloxy group, a biphenylyloxy group, a terphenylyloxy group, a naphthyloxy group, an anthryloxy group, a phenanthryloxy group, a fluorenyloxy group, and an indenyloxy group.

Specific examples of the "linear or branched cycloalkoxy group having 3 to 10 carbon atoms" in the "linear or branched cycloalkoxy group having 3 to 10 carbon atoms which may have a substituent" represented by $R^3$ to $R^{22}$ in the general formula (1) include a cyclopropoxy group, a cyclobutoxy group, a cyclopentyloxy group, a cyclohexyloxy group, and a 4-methylcyclohexyloxy group.

Examples of the "acyl group having 1 to 20 carbon atoms" in the "acyl group having 1 to 20 carbon atoms which may have a substituent" represented by $R^3$ to $R^{22}$ in the general formula (1) are the same as those of the "acyl group having 1 to 20 carbon atoms which may have a substituent" represented by $R^1$ and $R^2$ in the general formula (1).

Examples of the "thio group having 1 to 18 carbon atoms" in the "thio group having 1 to 18 carbon atoms which may have a substituent" represented by $R^3$ to $R^{22}$ in the general formula (1) are the same as those of the "thio group having 1 to 18 carbon atoms which may have a substituent" represented by $R^1$ and $R^2$ in the general formula (1).

Examples of the "amino group having 1 to 20 carbon atoms" in the "amino group having 1 to 20 carbon atoms which may have a substituent" represented by $R^3$ to $R^{22}$ in the general formula (1) are the same as those of the "amino group having 1 to 20 carbon atoms which may have a substituent" represented by $R^1$ and $R^2$ in the general formula (1).

Specific examples of the "aromatic hydrocarbon group having 6 to 36 carbon atoms" in the "aromatic hydrocarbon group having 6 to 36 carbon atoms which may have a substituent" represented by $R^3$ to $R^{22}$ in the general formula (1) include a phenyl group, a biphenyl group, a terphenyl group, a naphthyl group, a biphenyl group, an anthracenyl group (anthryl group), a phenanthryl group, a fluorenyl group, an indenyl group, a pyrenyl group, a perylenyl group, a fluoranthenyl group, and a triphenylenyl group. In the present invention, the aromatic hydrocarbon group encompasses a "condensed polycyclic aromatic group".

Specific examples of the "heterocyclic group having 5 to 36 ring atoms" in the "heterocyclic group having 5 to 36 ring atoms which may have a substituent" represented by $R^3$ to $R^{22}$ in the general formula (1) include a pyridyl group, a pyrimidinyl group, a triazinyl group, a thienyl group, a furyl group (furanyl group), a pyrrolyl group, an imidazolyl group, a pyrazolyl group, a triazolyl group, a quinolyl group, an isoquinolyl group, a naphthylidinyl group, an acridinyl group, a phenanthrolinyl group, a benzofuranyl group, a benzothienyl group, an oxazolyl group, an indolyl group, a carbazolyl group, a benzoxazolyl group, a thiazolyl group, a benzothiazolyl group, a quinoxalinyl group, a benzimidazolyl group, a pyrazolyl group, a dibenzofuranyl group, a dibenzothienyl group, and a carbonyl group.

Examples of the "substituent" in the "linear or branched alkyl group having 1 to 18 carbon atoms which may have a substituent", the "linear or branched alkenyl group having 2 to 20 carbon atoms which may have a substituent", the "linear or branched alkynyl group having 2 to 20 carbon atoms which may have a substituent", the "cycloalkyl group having 3 to 10 carbon atoms which may have a substituent", the "alkoxy group having 1 to 20 carbon atoms which may have a substituent", the "linear or branched cycloalkoxy group having 3 to 10 carbon atoms which may have a substituent", the "acyl group having 1 to 20 carbon atoms which may have a substituent", the "thio group having 1 to 18 carbon atoms which may have a substituent", the "amino group having 1 to 20 carbon atoms which may have a substituent", the "aromatic hydrocarbon group having 6 to 36 carbon atoms which may have a substituent", or the "heterocyclic group having 5 to 36 ring atoms which may have a substituent" represented by $R^3$ to $R^{22}$ in the general formula (1) are the same as those of the "substituent" in the "linear or branched acyl group having 1 to 18 carbon atoms which may have a substituent" or the like represented by $R^1$ and $R^2$ in the general formula (1).

In the general formula (1), $R^3$ to $R^7$, $R^8$ to $R^{12}$, $R^{13}$ to $R^{17}$, and $R^{18}$ to $R^{22}$ may be bonded to each other between adjacent groups to form a ring, and $R^7$ and $R^8$, and $R^{17}$ and $R^{18}$ may be bonded to each other by a single bond, a bond via an oxygen atom, a sulfur atom, or a selenium atom, or a bond via a nitrogen atom to form a ring. In the case of forming a ring, $R^7$ and $R^8$, and $R^{17}$ and $R^{18}$ are preferably bonded to each other by a single bond, or a bond via an oxygen atom or a sulfur atom to form a ring.

In the general formula (1), when $R^1$ and $R^2$ are a nitrile group, and $R^5$ is a methoxy group, $R^{10}$, $R^{15}$, and $R^{20}$ are at least one of the above groups other than a methoxy group.

In the general formula (1), $R^1$ and $R^2$ are each independently preferably a nitrile group, a linear or branched

acyl group having 1 to 18 carbon atoms which may have a substituent, or a linear or branched alkoxycarbonyl group having 1 to 18 carbon atoms which may have a substituent. $R^1$ and $R^2$ may be a nitrile group. $R^1$ may be a nitrile group, and $R^2$ may be a linear or branched alkoxycarbonyl group having 1 to 18 carbon atoms which may have a substituent. $R^1$ and $R^2$ may be a linear or branched alkoxycarbonyl group having 1 to 18 carbon atoms which may have a substituent. $R^1$ and $R^2$ may be a linear or branched acyl group having 1 to 18 carbon atoms which may have a substituent, and may be bonded to each other to form a ring.

In the general formula (1), $R^3$ to $R^{22}$ are preferably a hydrogen atom, a linear or branched alkoxy group having 1 to 20 carbon atoms which may have a substituent, or an amino group having 1 to 20 carbon atoms which may have a substituent. $R^5$, $R^{10}$, $R^{15}$, and $R^{20}$ may be a linear or branched alkoxy group having 1 to 20 carbon atoms which may have a substituent, and may be, for example, a methoxy group. $R^5$ and $R^{15}$ may be a linear or branched alkoxy group having 1 to 20 carbon atoms which may have a substituent, $R^{10}$ and $R^{20}$ may be an amino group having 1 to 20 carbon atoms which may have a substituent, and the amino group may include a linear or branched alkoxy group having 1 to 20 carbon atoms. $R^5$, $R^{10}$, $R^{15}$, and $R^{20}$ may be an amino group having 1 to 20 carbon atoms which may have a substituent, and the amino group may include a linear or branched alkoxy group having 1 to 20 carbon atoms. $R^7$ and $R^8$, and $R^{17}$ and $R^{18}$ may be bonded to each other by a single bond, or a bond via an oxygen atom or a sulfur atom to form a ring.

In the general formula (1), $X^1$ and $X^2$ are preferably represented by the general formula (2).

In the general formula (2), $R^{23}$ to $R^{28}$ each independently represent

a hydrogen atom,
a linear or branched alkyl group having 1 to 20 carbon atoms which may have a substituent,
a linear or branched alkenyl group having 2 to 20 carbon atoms which may have a substituent,
a linear or branched alkynyl group having 2 to 20 carbon atoms which may have a substituent,
a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms which may have a substituent, or
a heterocyclic group having 5 to 36 ring atoms which may have a substituent.

Examples of the "linear or branched alkyl group having 1 to 20 carbon atoms" in the "linear or branched alkyl group having 1 to 20 carbon atoms which may have a substituent" represented by $R^{13}$ to $R^{28}$ in the general formula (2) are the same as those of the "linear or branched alkyl group having 1 to 20 carbon atoms which may have a substituent" represented by $R^3$ to $R^{22}$ in the general formula (1).

Examples of the "linear or branched alkenyl group having 2 to 20 carbon atoms" in the "linear or branched alkenyl group having 2 to 20 carbon atoms which may have a substituent" represented by $R^{23}$ to $R^{28}$ in the general formula (2) are the same as those of the "linear or branched alkenyl group having 2 to 20 carbon atoms which may have a substituent" represented by $R^3$ to $R^{22}$ in the general formula (1).

Examples of the "linear or branched alkynyl group having 2 to 20 carbon atoms" in the "linear or branched alkynyl group having 2 to 20 carbon atoms which may have a substituent" represented by $R^{23}$ to $R^{28}$ in the general formula (2) are the same as those of the "linear or branched alkynyl group having 2 to 20 carbon atoms which may have a substituent" represented by $R^3$ to $R^{22}$ in the general formula (1).

Examples of the "cycloalkyl group having 3 to 10 carbon atoms" in the "cycloalkyl group having 3 to 10 carbon atoms which may have a substituent" represented by $R^{13}$ to $R^{28}$ in the general formula (2) are the same as those of the "cycloalkyl group having 3 to 10 carbon atoms which may have a substituent" represented by $R^3$ to $R^{22}$ in the general formula (1).

Examples of the "linear or branched alkoxy group having 1 to 20 carbon atoms" in the "linear or branched alkoxy group having 1 to 20 carbon atoms which may have a substituent" represented by $R^{13}$ to $R^{28}$ in the general formula (2) are the same as those of the "linear or branched alkoxy group having 1 to 20 carbon atoms which may have a substituent" represented by $R^3$ to $R^{22}$ in the general formula (1).

Examples of the "cycloalkoxy group having 3 to 10 carbon atoms" in the "cycloalkoxy group having 3 to 10 carbon atoms which may have a substituent" represented by $R^{23}$ to $R^{28}$ in the general formula (2) are the same as those of the "cycloalkoxy group having 3 to 10 carbon atoms which may have a substituent" represented by $R^3$ to $R^{22}$ in the general formula (1).

Examples of the "thio group having 1 to 18 carbon atoms" in the "thio group having 1 to 18 carbon atoms which may have a substituent" represented by $R^{23}$ to $R^{28}$ in the general formula (2) are the same as those of the "thio group having 1 to 18 carbon atoms which may have a substituent" represented by $R^1$ and $R^2$ in the general formula (1).

Examples of the "amino group having 1 to 20 carbon atoms" in the "amino group having 1 to 20 carbon atoms which may have a substituent" represented by $R^{23}$ to $R^{28}$ in the general formula (2) are the same as those of the "amino group having 1 to 20 carbon atoms which may have a substituent" represented by $R^1$ and $R^2$ in the general formula (1).

Examples of the "aromatic hydrocarbon group having 6 to 36 carbon atoms" in the "aromatic hydrocarbon group having 6 to 36 carbon atoms which may have a substituent" represented by $R^{23}$ to $R^{28}$ in the general formula (2) are the same as those of the "aromatic hydrocarbon group having 6 to 36 carbon atoms which may have a substituent" represented by $R^3$ to $R^{22}$ in the general formula (1).

Examples of the "heterocyclic group having 5 to 36 ring atoms" in the "heterocyclic group having 5 to 36 ring atoms which may have a substituent" represented by $R^{23}$ to $R^{28}$ in the general formula (2) are the same as those of the "heterocyclic group having 5 to 36 ring atoms which may have a substituent" represented by $R^3$ to $R^{22}$ in the general formula (1).

Examples of the "substituent" in the "linear or branched alkyl group having 1 to 18 carbon atoms which may have a substituent", the "linear or branched alkenyl group having 2 to 20 carbon atoms which may have a substituent", the "linear or branched alkynyl group having 2 to 20 carbon atoms which may have a substituent", the "cycloalkyl group having 3 to 10 carbon atoms which may have a substituent", the "alkoxy group having 1 to 20 carbon atoms which may have a substituent", the "linear or branched cycloalkoxy group having 3 to 10 carbon atoms which may have a substituent", the "acyl group having 1 to 20 carbon atoms which may have a substituent", the "thio group having 1 to 18 carbon atoms which may have a substituent", the "amino group having 1 to 20 carbon atoms which may have a substituent", the "aromatic hydrocarbon group having 6 to 36 carbon atoms which may have a substituent", or the "heterocyclic group having 5 to 36 ring atoms which may have a substituent" represented by $R^{23}$ to $R^{28}$ in the general formula (2) are the same as those of the "substituent" in the "linear or branched acyl group having 1 to 18 carbon atoms which may have a substituent" or the like represented by $R^1$ and $R^2$ in the general formula (1).

In the general formula (2), $R^{23}$ and $R^{24}$, $R^{25}$ and $R^{26}$, and $R^{27}$ and $R^{28}$ may be bonded to each other by a single bond, a bond via an oxygen atom, a sulfur atom, or a selenium atom, or a bond via a nitrogen atom to form a ring.

In the general formula (2), $Y^1$ represents an oxygen atom, a sulfur atom, or a selenium atom.

In the general formula (2), m and n each represent an integer of 0 to 2. When m is 0, n is set to be 1 to 2, and when n is 0, m is set to be 1 to 2. Any one of m and n is 1 or more. The case where both m and n are 0 is not included. m is preferably 1. A cyclopentadithiophene moiety which is a central skeleton of the general formula (1) may be bonded to either a phenyl group or a 5-membered heterocyclic group represented by the general formula (2).

In the general formula (2), m is preferably 1, and n is preferably 0 or 1. In the general formula (2), when m is 1 and n is 1, a 5-membered heterocyclic group is preferably bonded to the cyclopentadithiophene moiety which is the central skeleton of the general formula (1).

Specific examples of the compound represented by the general formula (1) in the present invention are shown below, but the present invention is not limited thereto. The following exemplified compounds are described with partial omission of hydrogen atoms, carbon atoms, and the like, and are examples of possible isomers, and include all other isomers. The compounds each may be a mixture of two or more isomers.

[Chem. 3]

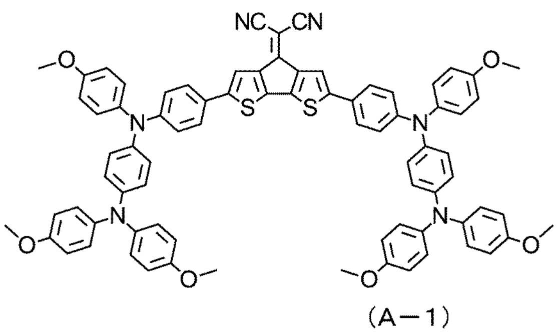

(A−1)

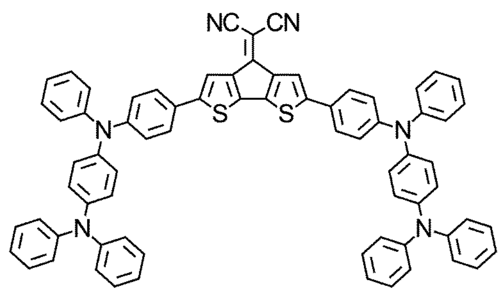

(A−2)

[Chem. 4]

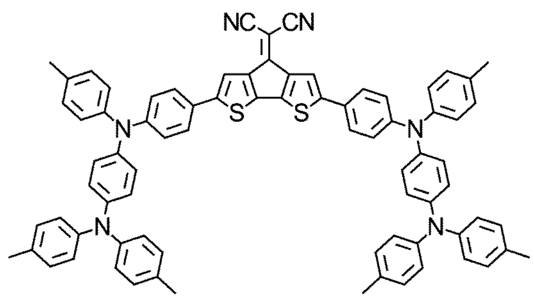

(A－3)

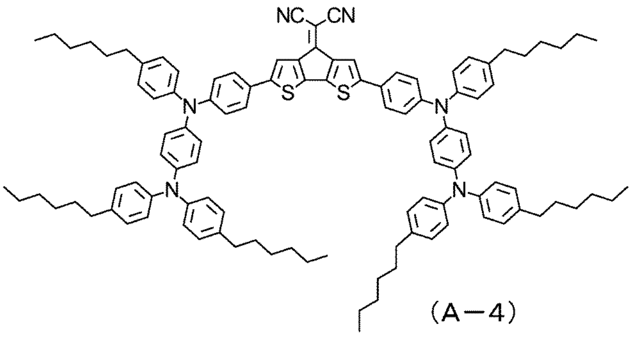

(A－4)

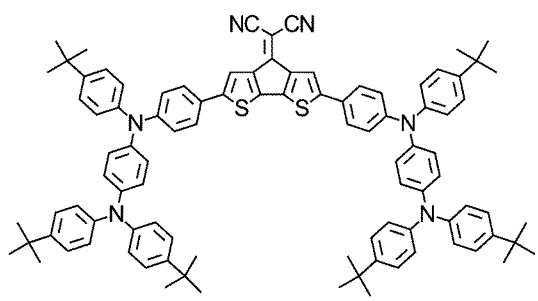

(A－5)

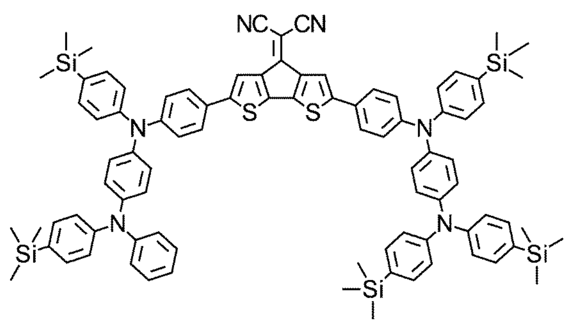

(A－6)

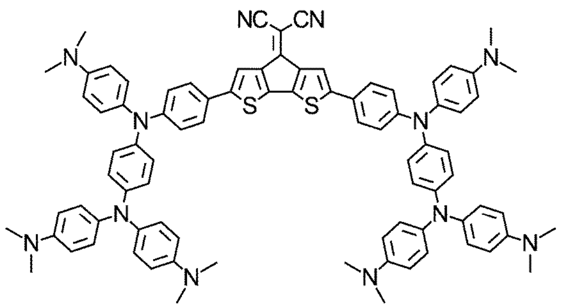

(A－7)

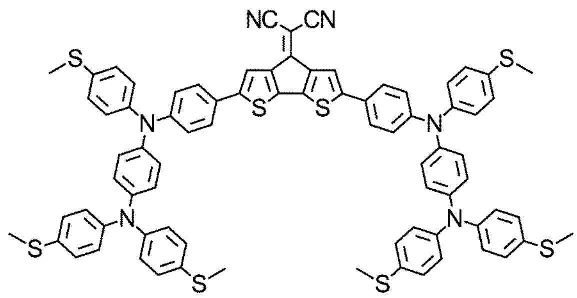

(A－8)

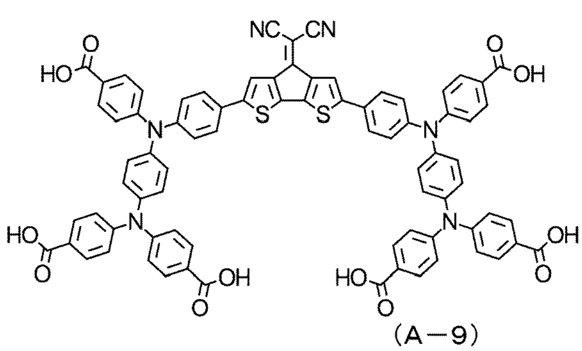

(A－9)

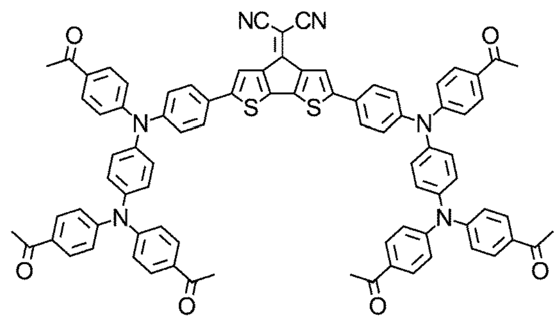

(A－10)

[Chem. 5]

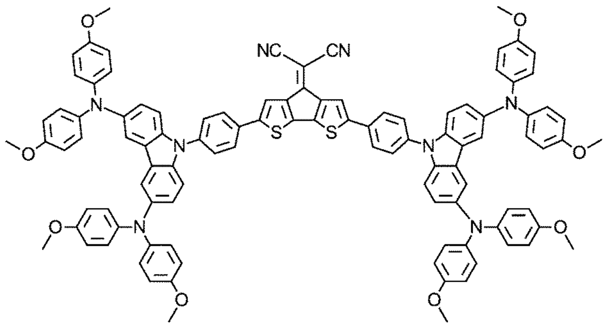

(A-11)

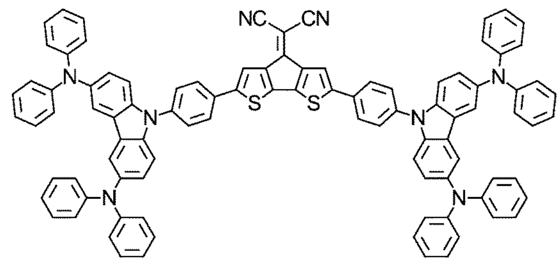

(A-12)

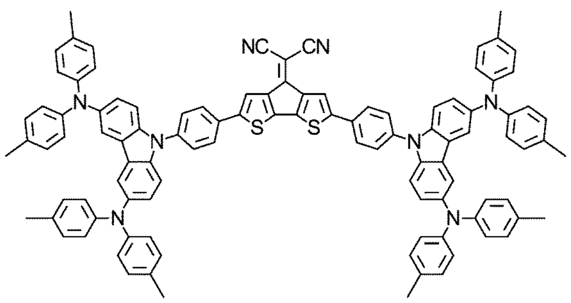

(A-13)

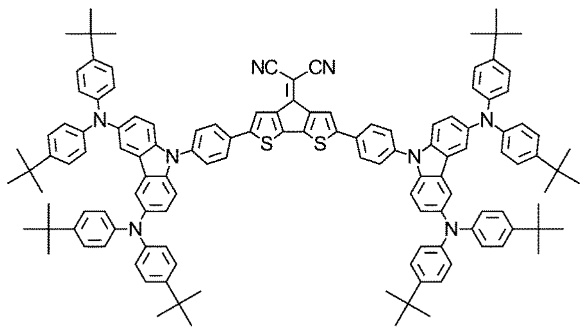

(A-14)

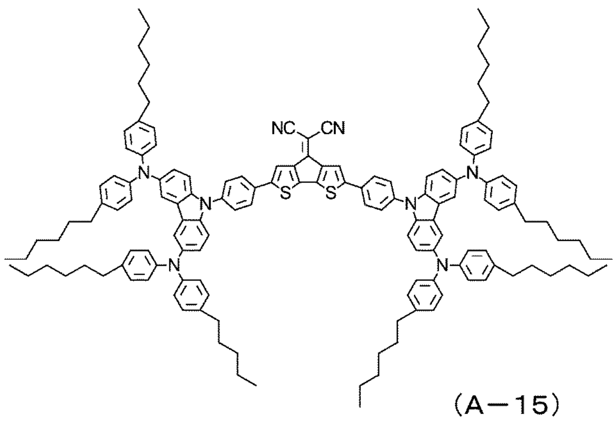

(A-15)

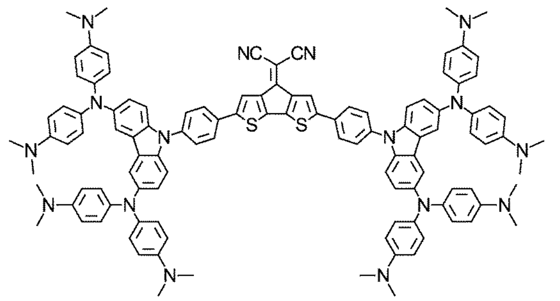

(A-16)

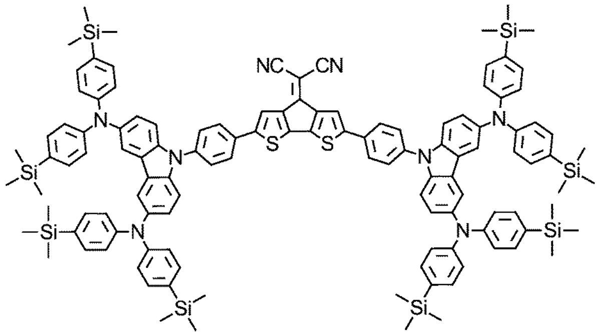

(A-17)

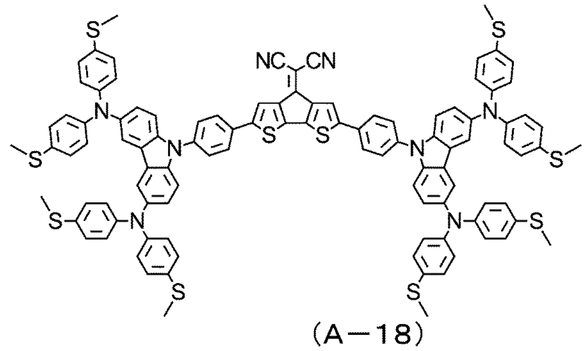

(A-18)

[Chem. 6]

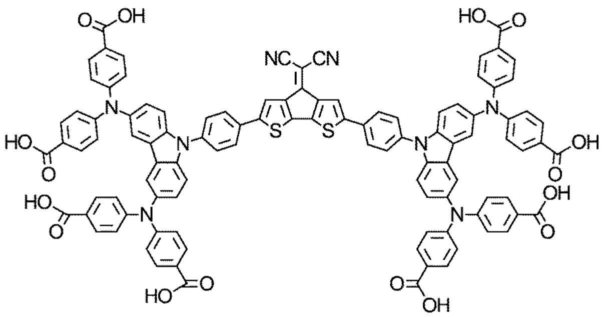

(A−19)

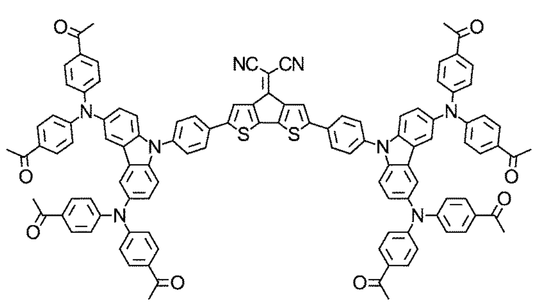

(A−20)

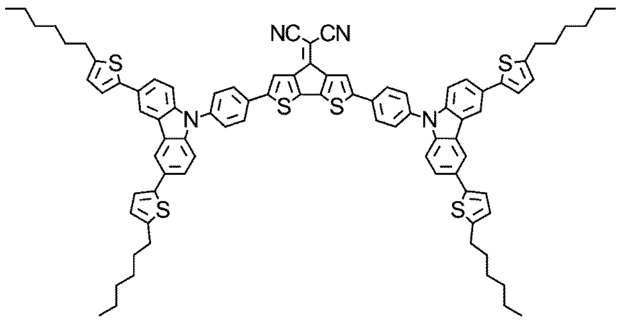

(A−21)

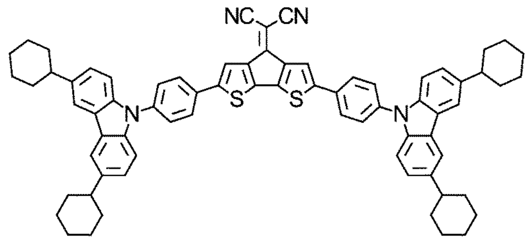

(A−22)

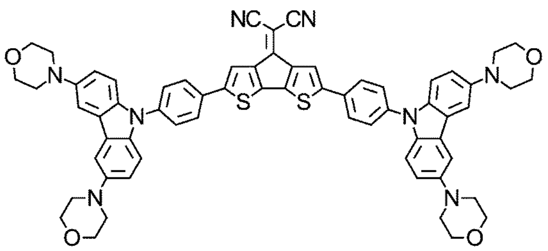

(A−23)

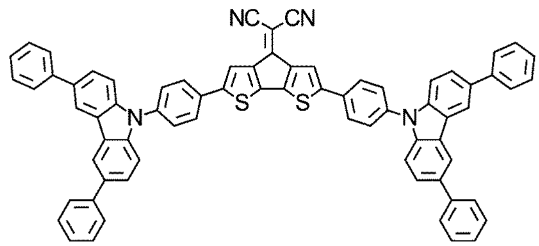

(A−24)

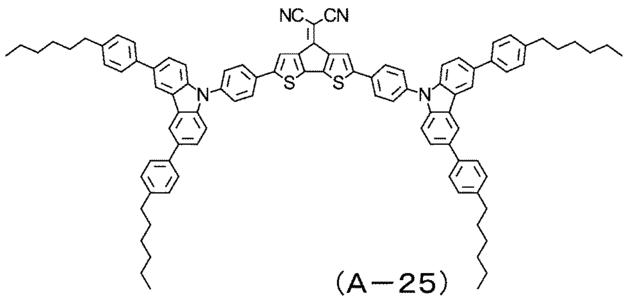

(A−25)

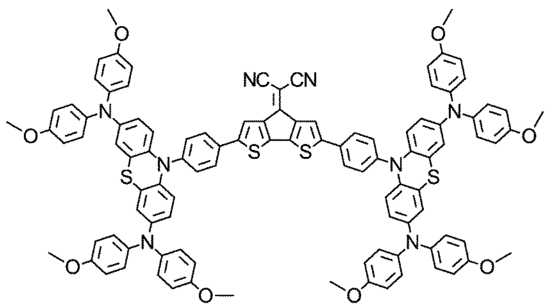

(A−26)

[Chem. 7]

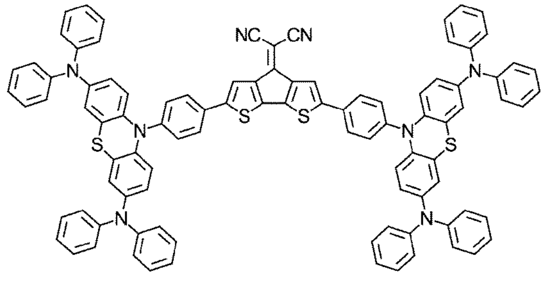

(A-27)

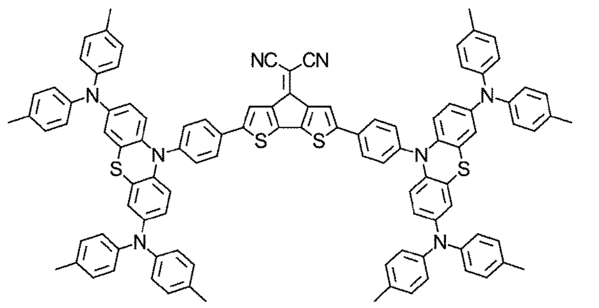

(A-28)

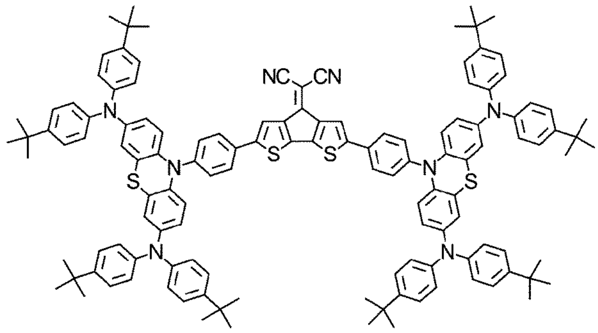

(A-29)

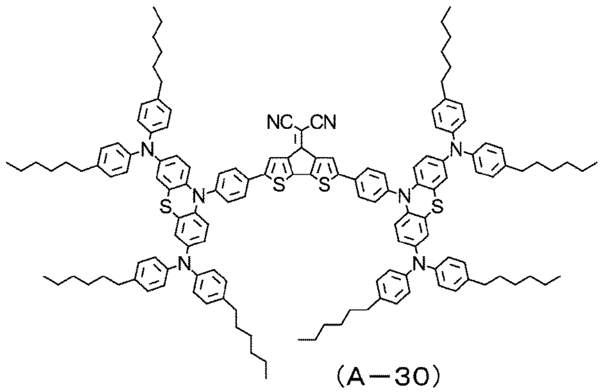

(A-30)

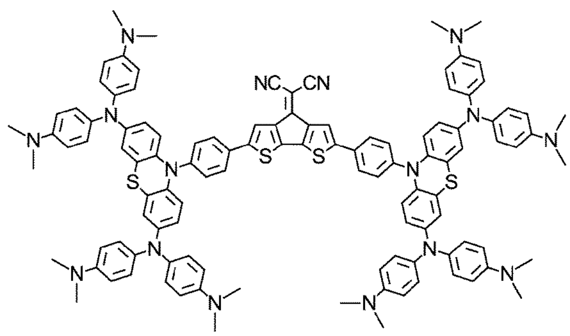

(A-31)

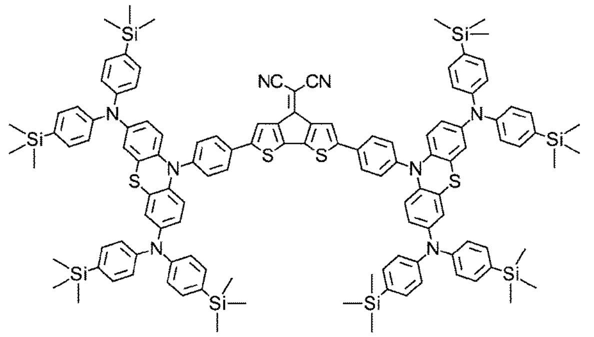

(A-32)

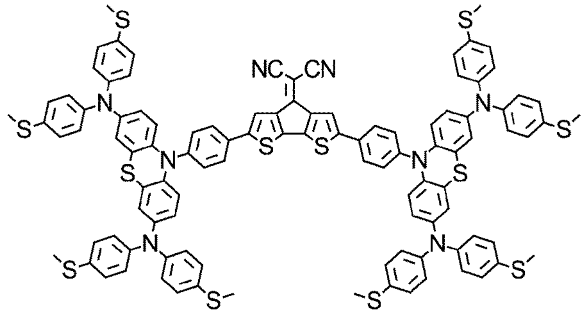

(A-33)

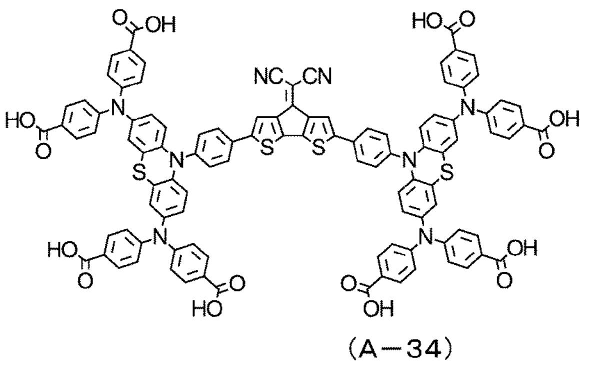

(A-34)

[Chem. 8]

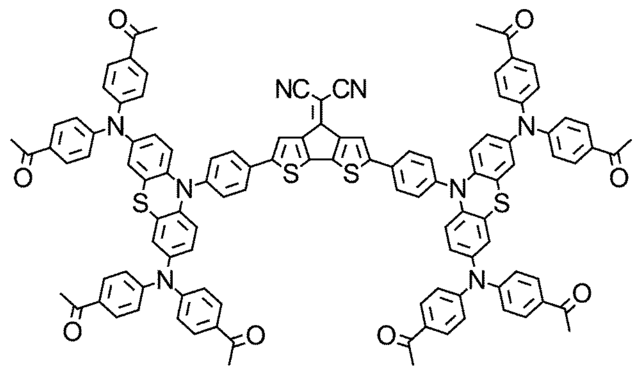

(A-35)

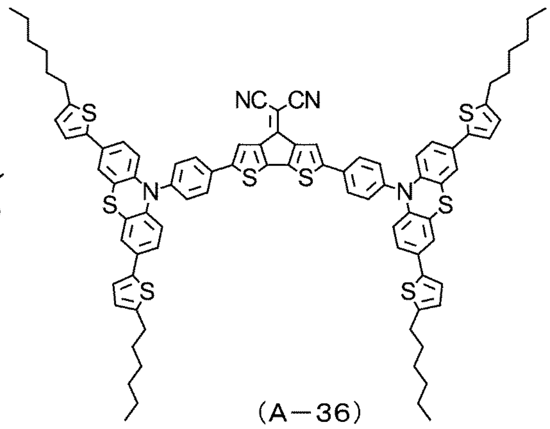

(A-36)

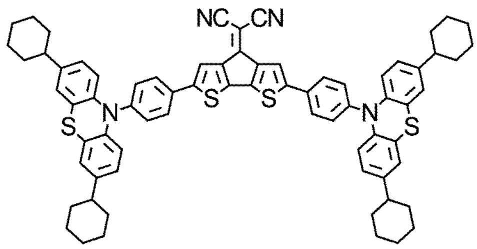

(A-37)

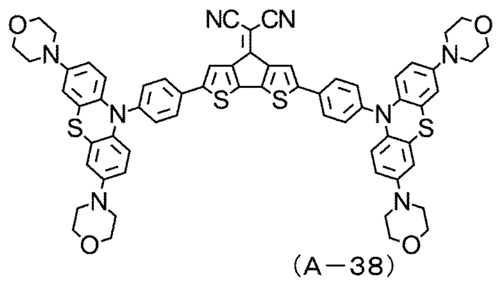

(A-38)

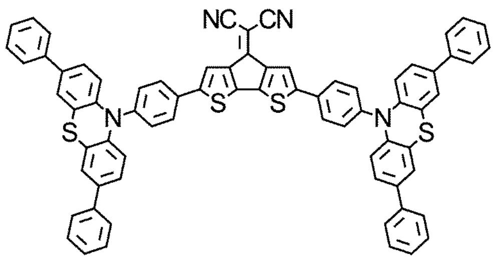

(A-39)

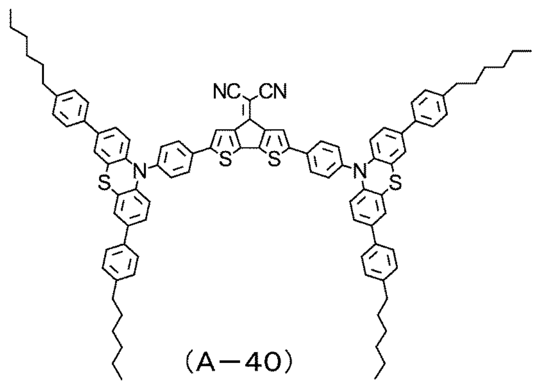

(A-40)

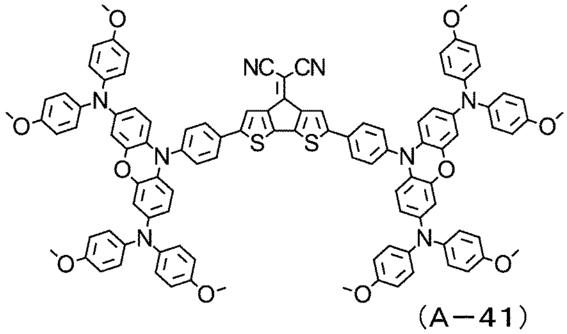

(A-41)

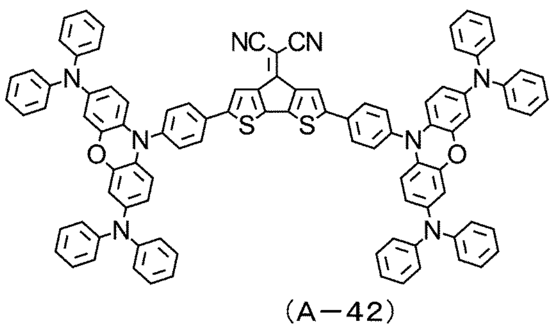

(A-42)

[Chem. 9]

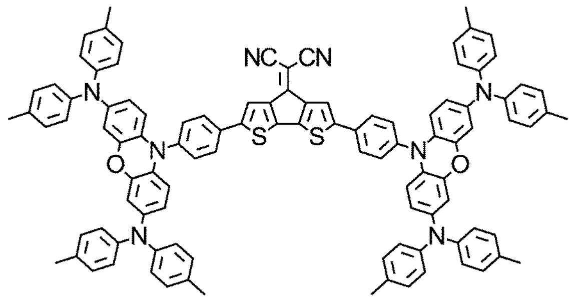

(A-43)

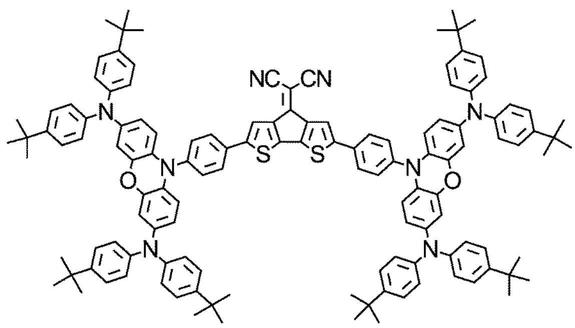

(A-44)

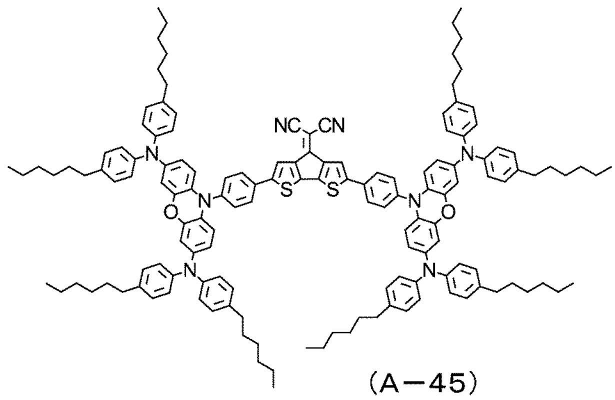

(A-45)

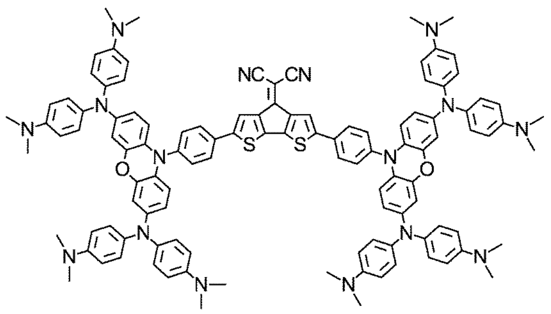

(A-46)

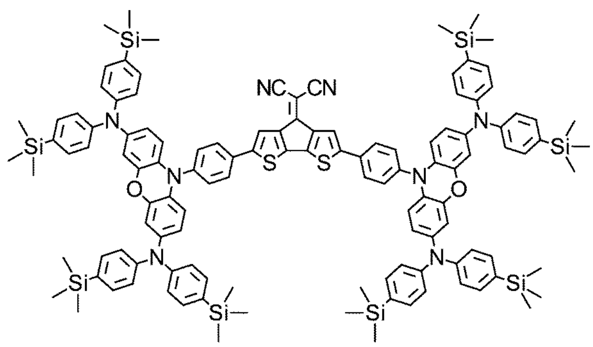

(A-47)

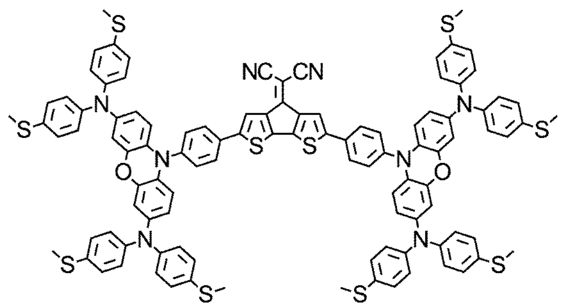

(A-48)

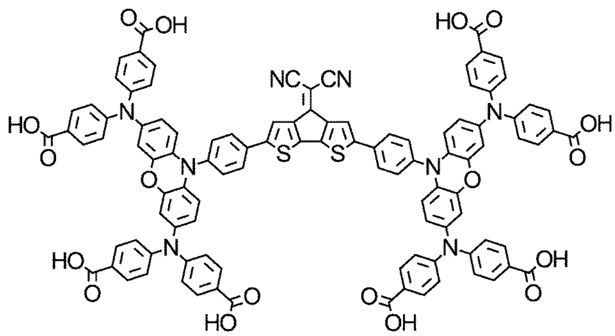

(A-49)

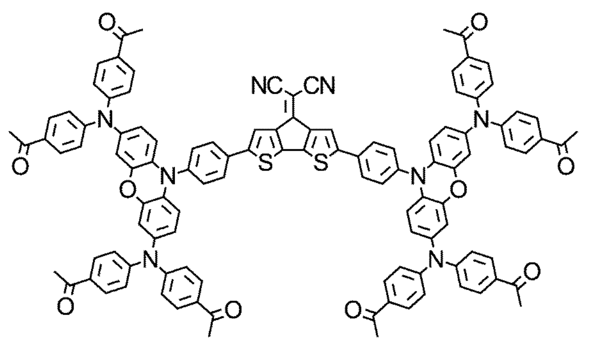

(A-50)

[Chem. 10]

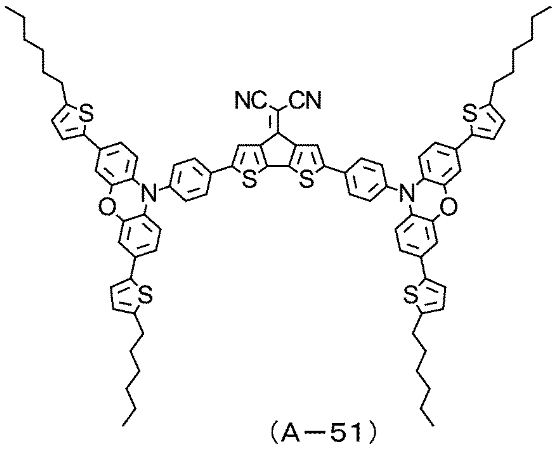

(A−51)

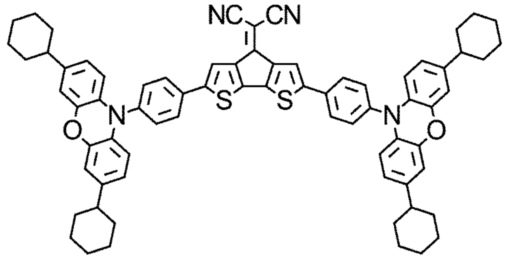

(A−52)

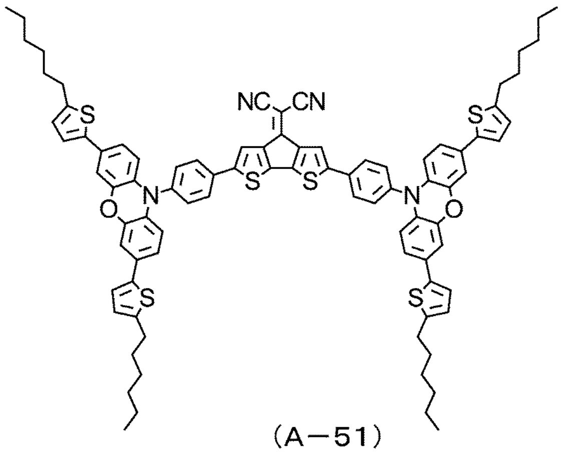

(A−51)

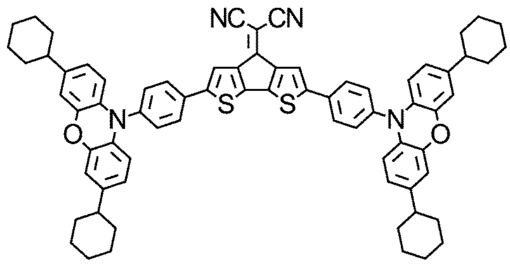

(A−52)

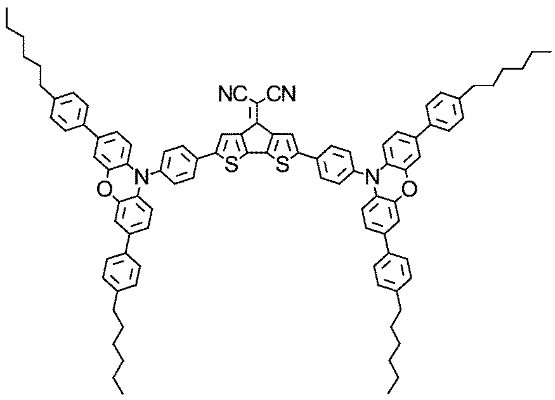

(A−55)

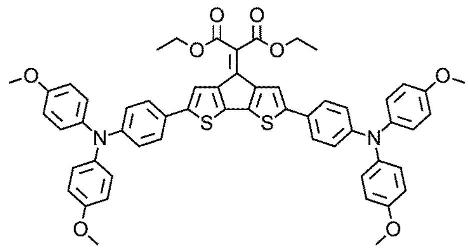

(A−56)

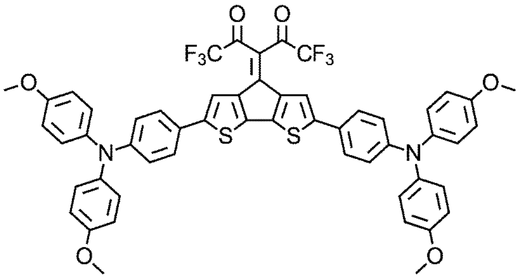

(A−57)

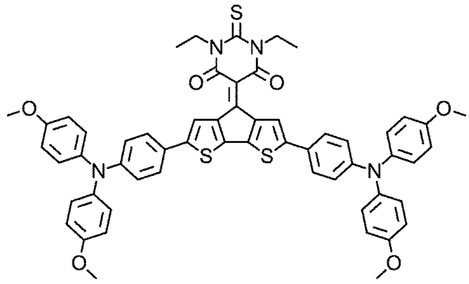

(A−58)

[Chem. 11]

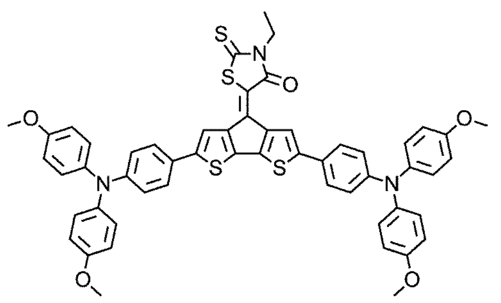

(A-59)

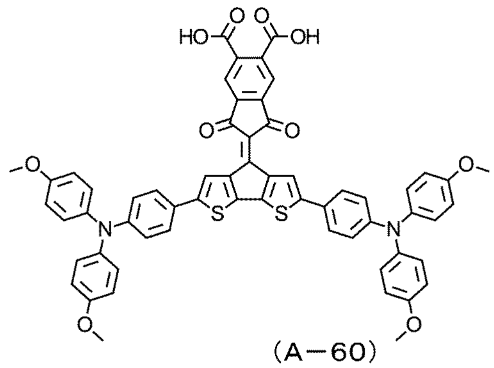

(A-60)

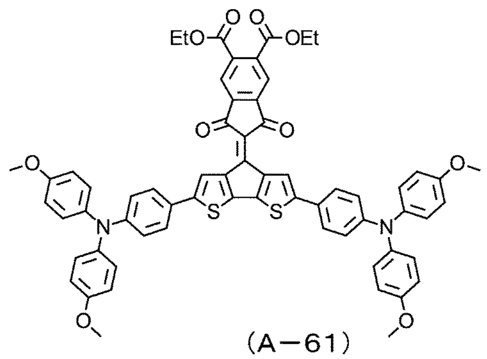

(A-61)

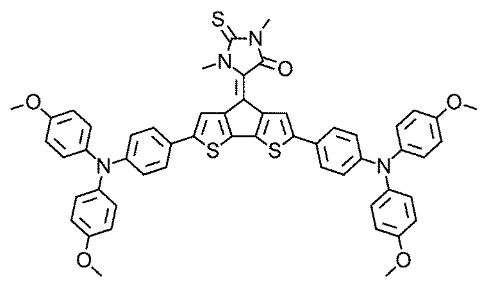

(A-62)

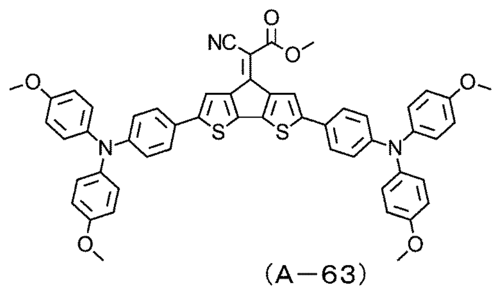

(A-63)

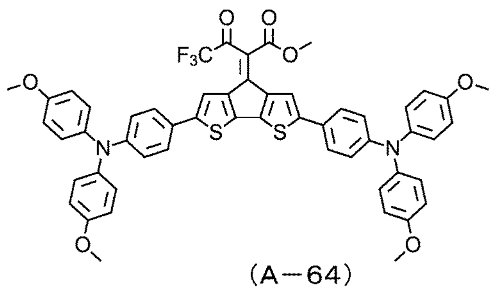

(A-64)

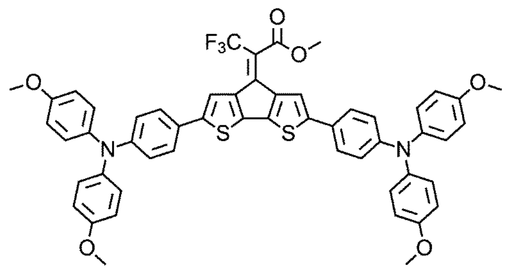

(A-65)

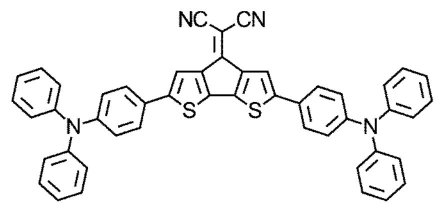

(A-66)

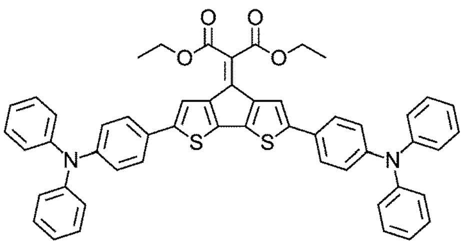

(A-67)

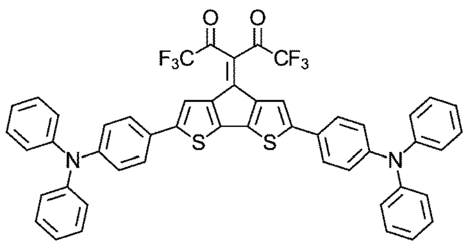

(A-68)

[Chem. 12]

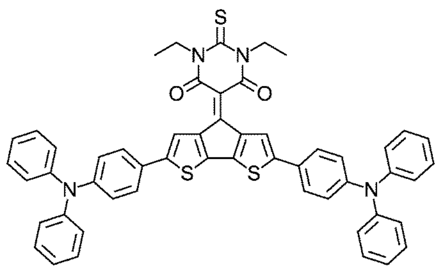

(A−69)

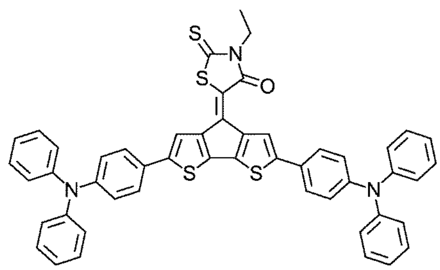

(A−70)

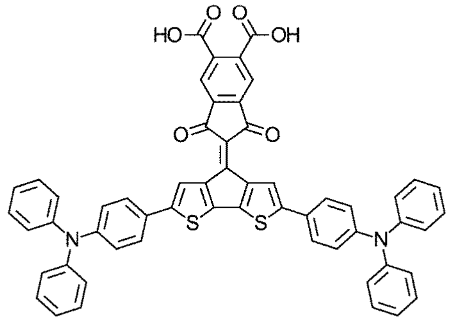

(A−71)

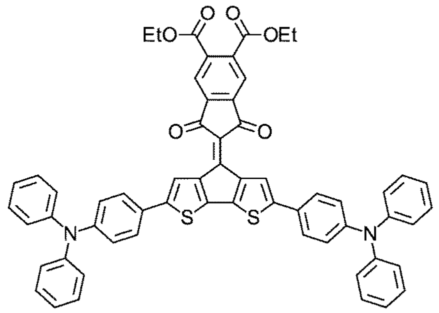

(A−72)

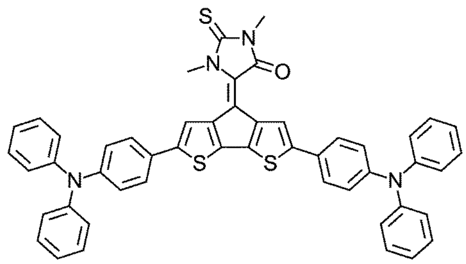

(A−73)

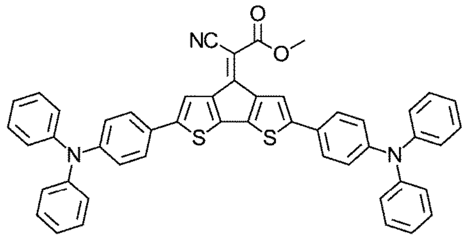

(A−74)

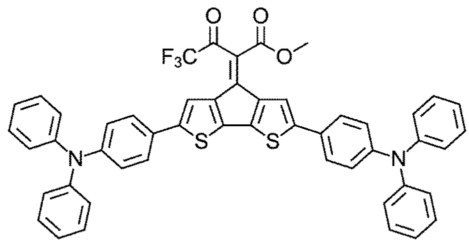

(A−75)

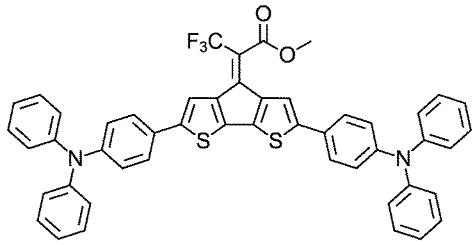

(A−76)

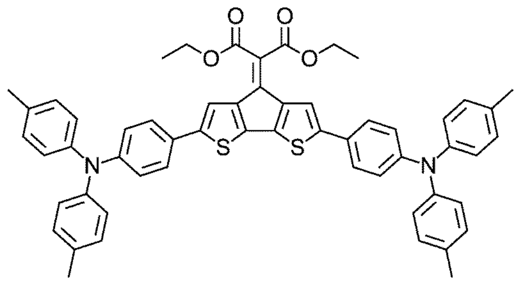

(A−77)

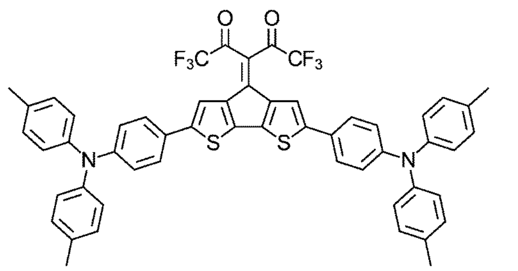

(A−78)

[Chem. 13]

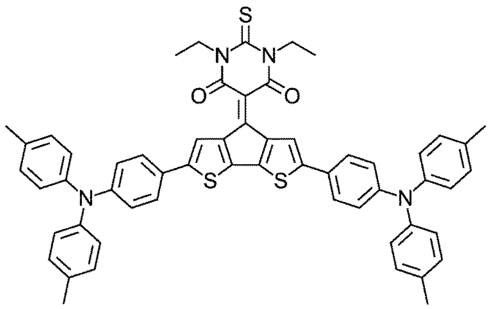

(A－79)

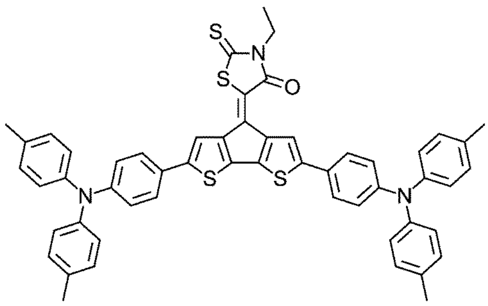

(A－80)

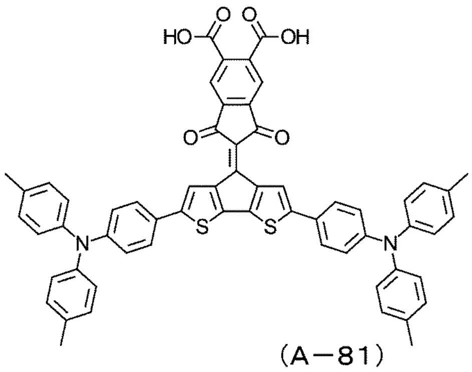

(A－81)

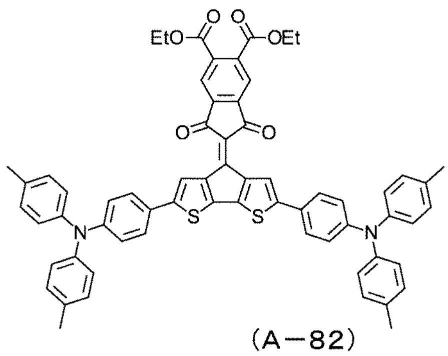

(A－82)

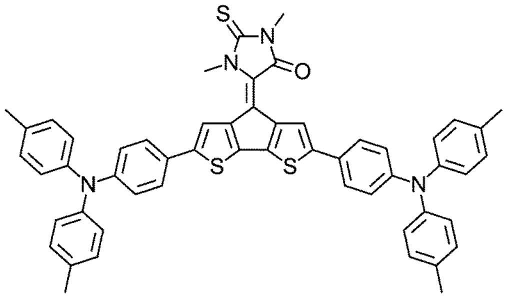

(A－83)

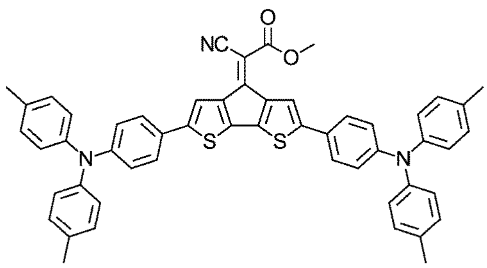

(A－84)

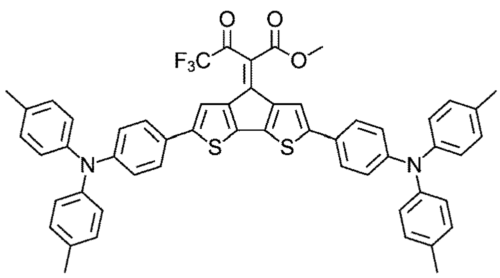

(A－85)

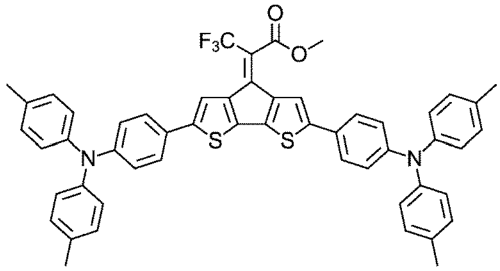

(A－86)

[Chem. 14]

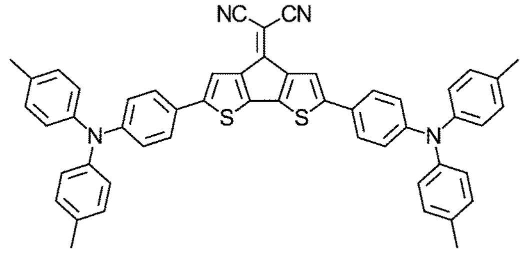

(A−87)

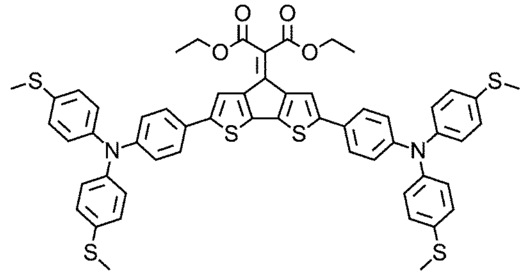

(A−88)

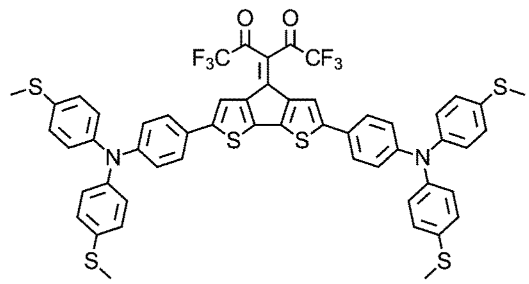

(A−89)

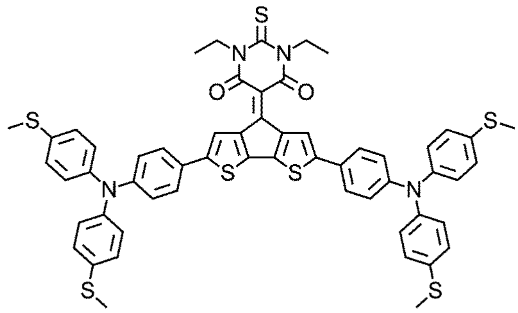

(A−90)

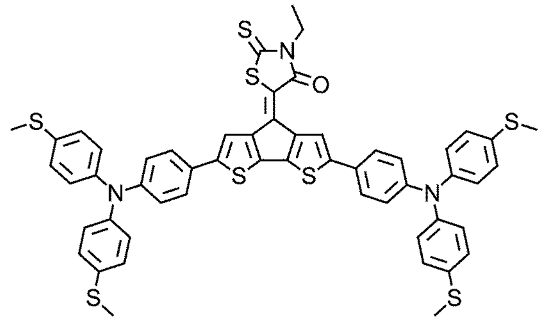

(A−91)

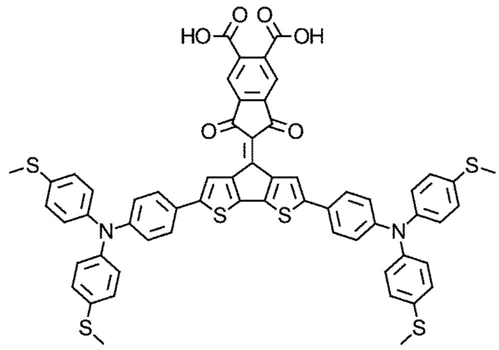

(A−92)

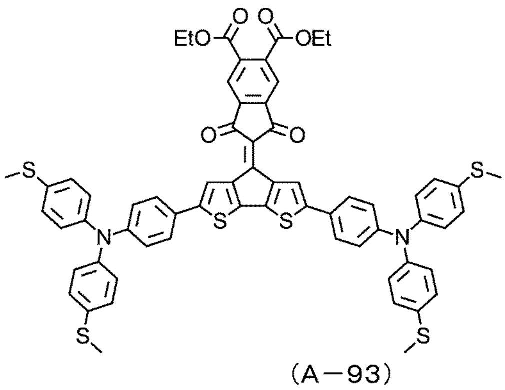

(A−93)

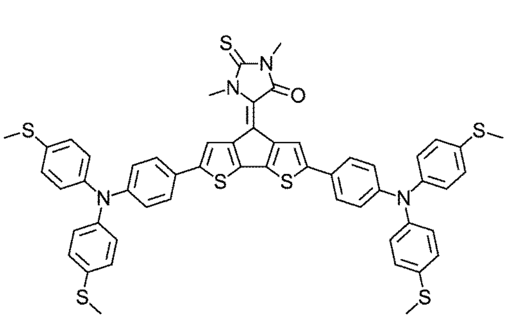

(A−94)

[Chem. 15]

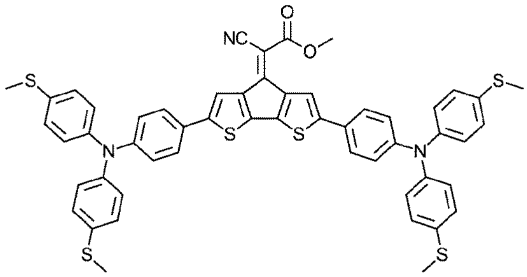

(A−95)

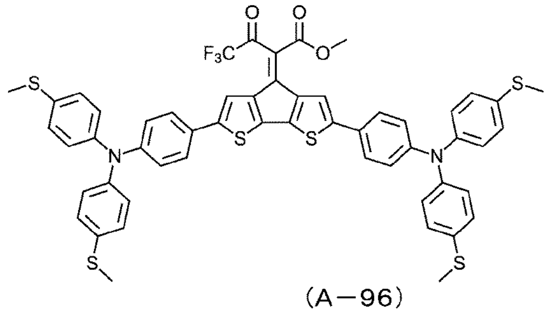

(A−96)

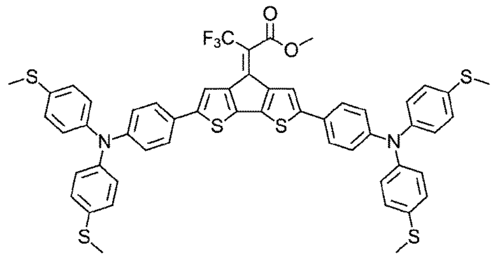

(A−97)

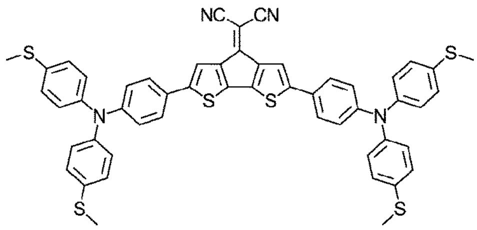

(A−98)

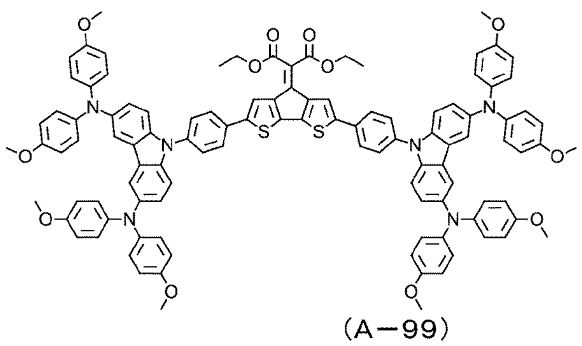

(A−99)

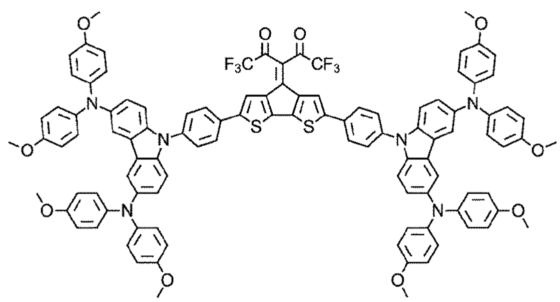

(A−100)

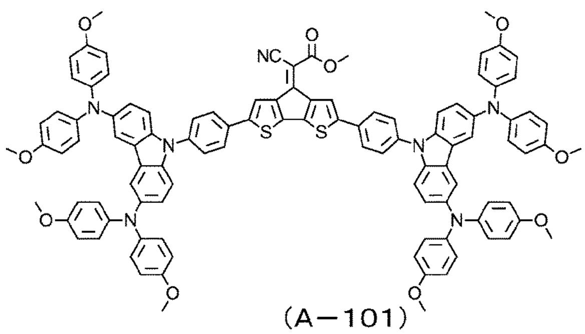

(A−101)

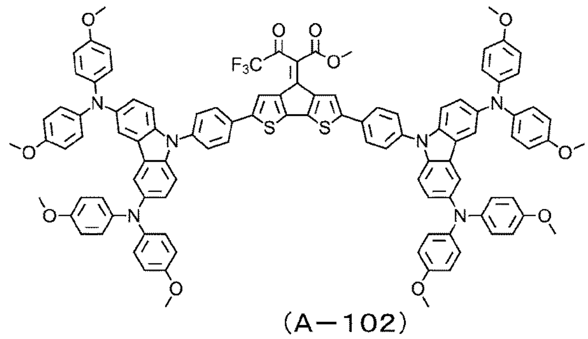

(A−102)

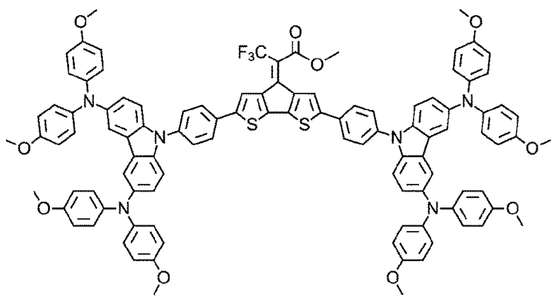

(A−103)

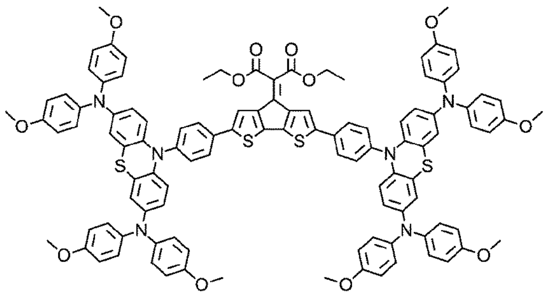

(A−104)

[Chem. 16]

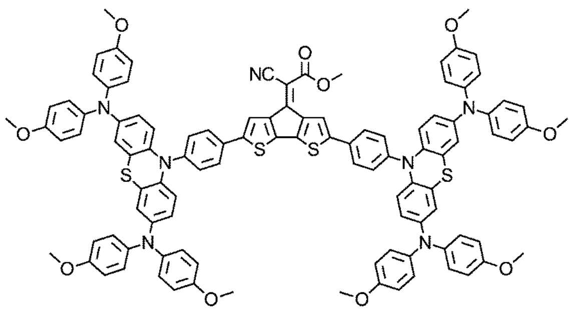

(A－105)

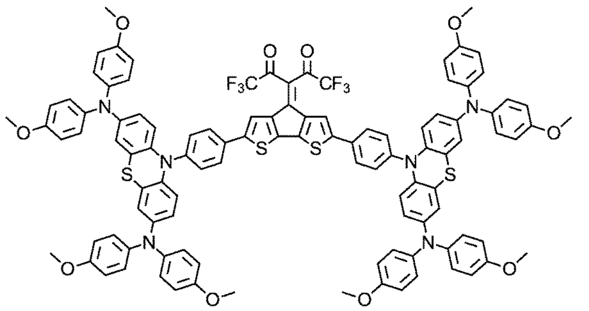

(A－106)

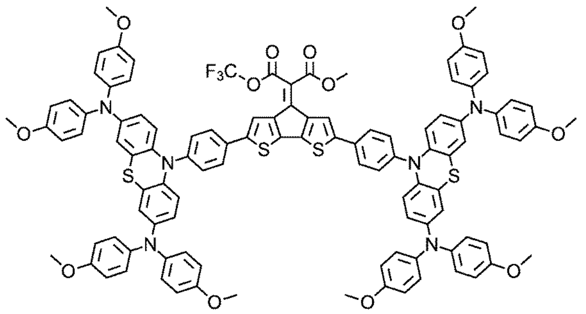

(A－107)

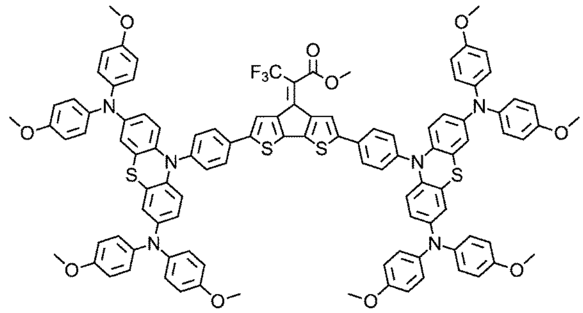

(A－108)

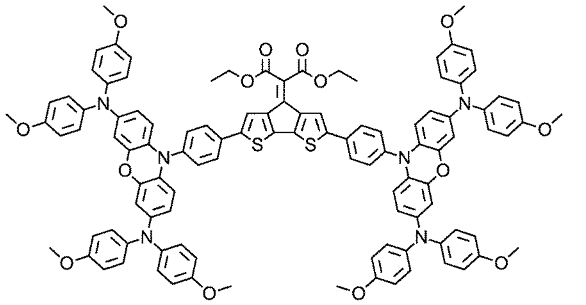

(A－109)

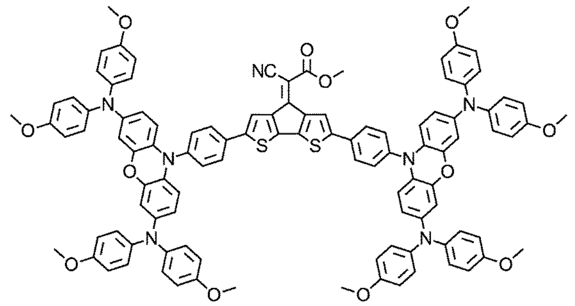

(A－110)

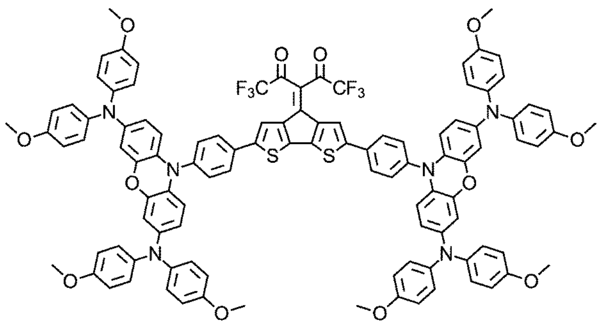

(A－111)

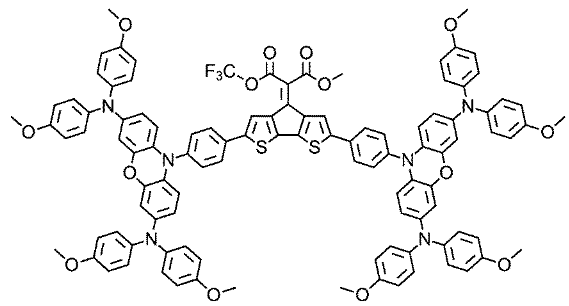

(A－112)

[Chem. 17]

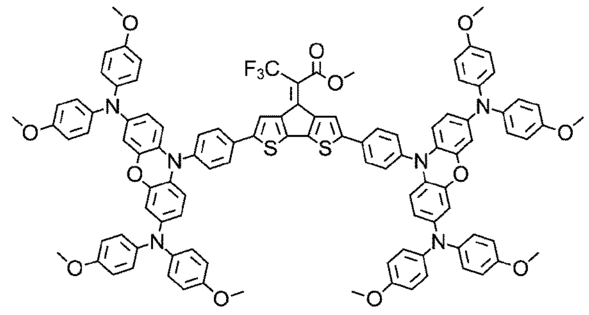

(A－１１３)

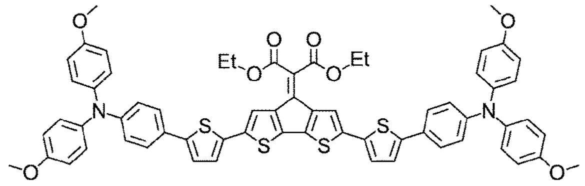

(A−114)

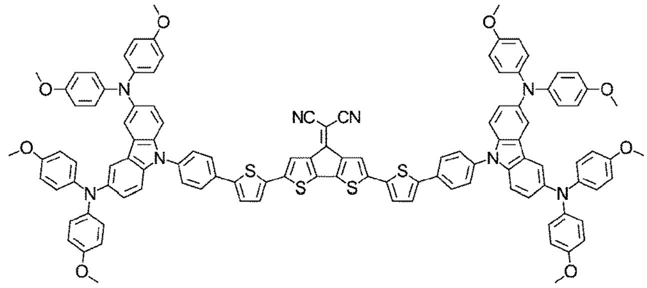

(A−115)

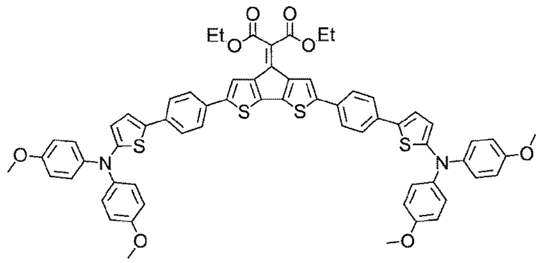

(A−116)

The hole transport material for a photoelectric conversion element according to the present invention represented by the general formula (1) can be synthesized by a known method.

For example, a compound in which $X^1$ and $X^2$ are represented by the general formula (2) where m = 1 and n = 0, such as a compound (A-1), can be synthesized by a Suzuki-Miyaura cross-coupling reaction between a dibromo compound represented by the following formula (3) and a boronic acid compound represented by the following general formulae (4) and (5) or a boronic acid ester compound represented by the following general formulae (6) and (7), and further by a Knoevenagel condensation reaction with a compound represented by the following general formula (8).

[Chem. 18]

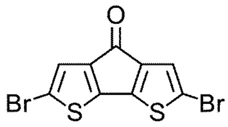

(3)

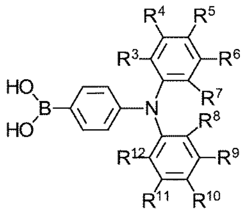

(4)

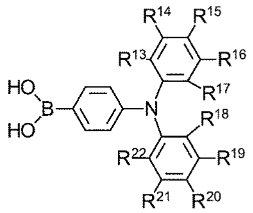

(5)

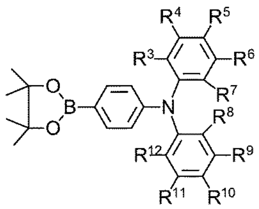

(6)

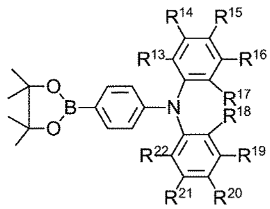

(7)

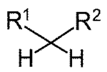

(8)

The compound represented by the general formula (1) in the present invention can be purified by column purification using column chromatography, adsorption purification using silica gel, activated carbon, activated clay, or the like, recrystallization or crystallization using a solvent, or the like. Alternatively, it is effective to use a compound having an increased purity by using these methods in combination. These compounds can be identified by nuclear magnetic resonance analysis (NMR).

Hereinafter, preferred aspects of the photoelectric conversion element according to the present invention will be described.

The photoelectric conversion element according to the present invention preferably has a configuration including the conductive support 1, the hole blocking layer 2, the electron transport layer 3, the photoelectric conversion layer 4, the hole transport layer 5, and the counter electrode 6 as illustrated in FIG. 1, or a configuration including the conductive support 7, the electron transport layer 8, the photoelectric conversion layer 9, the hole transport layer 10, and the counter electrode 11 as illustrated in FIG. 2, but is not limited thereto. The photoelectric conversion element according to the present invention is preferably a perovskite photoelectric conversion element. In the present invention, the perovskite photoelectric conversion element preferably includes the conductive support 1, the hole blocking layer 2, the electron transport layer 3, the photoelectric conversion layer (perovskite layer) 4, the hole transport layer 5, and the counter electrode 6 in FIG. 1 in this order, or preferably includes the conductive support 7, the electron transport layer 8, the photoelectric conversion layer (perovskite layer) 9, the hole transport layer 10, and the counter electrode 11 in FIG. 2 in this order, but the electron transport layer may be omitted. The conductive support, the hole transport layer, the photoelectric conversion layer (perovskite layer), the electron transport layer, and the counter electrode may be formed in this order.

<Conductive Supports

In the present invention, the conductive support is required to have translucency allowing transmission of light that contributes to photoelectric conversion. The conductive support is a member having a function of extracting a current from the photoelectric conversion layer, and thus is preferably a conductive substrate. Specific examples of a conductive material include a conductive transparent oxide semiconductor such as tin-doped indium oxide (ITO), fluorine-doped tin oxide (FTO), and indium-tin composite oxide, and a glass substrate coated with a tin-doped indium oxide (ITO) or fluorine-doped tin oxide (FTO) thin film is preferably used.

<Hole Blocking Layer>

In the photoelectric conversion element according to the present invention, the hole blocking layer 2 is preferably formed on the conductive support 1 as illustrated in FIG. 1. Specific examples of a semiconductor of the hole blocking layer include one or more of titanium oxide (TiOz or the like), tungsten oxide ($WO_2$, $WO_3$, $W_2O_3$ or the like), zinc oxide (ZnO), niobium oxide ($Nb_2O_5$ or the like), tantalum oxide ($Ta_2O_5$ or the like), yttrium oxide ($Y_2O_3$ or the like), strontium titanate ($SrTiO_3$ or the like), a fullerene derivative, a carbon nanotube, and the like. In the present invention, titanium oxide is preferable.

A thickness of the hole blocking layer is not particularly limited, and is preferably about 5 nm to 100 nm, and more preferably about 30 nm to 90 nm, from a viewpoint of further preventing the photoelectric conversion layer from coming into contact with the conductive support and short-circuiting.

The hole blocking layer can be obtained by using a known film forming method depending on a material to be molded. Examples of a production method of the hole blocking layer include, but are not limited to, a spin coating method of a dispersion containing semiconductor fine particles, a spray pyrolysis method of spraying a precursor solution of a semiconductor onto a conductive support placed on a heated hot plate by spraying, and an ALD method of vaporizing a precursor to react and deposit the precursor. In the present invention, a commercially available product may be used as the dispersion containing semiconductor fine particles.

In the case where a commercially available dispersion containing semiconductor fine particles is used, a dry atmosphere is preferable from a viewpoint of producing a perovskite solar cell with good reproducibility and high efficiency by preventing aggregation of semiconductor fine particles due to moisture in an atmosphere.

<Electron Transport Layer>

In the photoelectric conversion element according to the present invention, the electron transport layer 3 is preferably formed on the hole blocking layer 2 as illustrated in FIG. 1, or the electron transport layer 8 is preferably formed on the conductive support 7 as illustrated in FIG. 2. An aspect is not particularly limited, and in the case of the configuration in FIG. 1, a thin film having a porous structure is preferable. By a porous structure, an active surface area of the photoelectric conversion layer can be significantly increased, a photoelectric conversion efficiency can be improved, and an electron transport layer excellent in electron collection can be obtained. The electron transport layer is used to improve an efficiency of movement of electrons from the photoelectric conversion layer to an electrode and to block movement of holes.

Specific examples of a semiconductor forming the electron transport layer include a metal oxide such as tin oxide (SnO, $SnO_2$, $SnO_3$, or the like), titanium oxide (TiOz or the like), tungsten oxide ($WO_2$, $WO_3$, $W_2O_3$, or the like), zinc oxide (ZnO), niobium oxide ($Nb_2O_5$ or the like), tantalum oxide ($Ta_2O_5$ or the like), yttrium oxide ($Y_2O_3$ or the like), and strontium titanate ($SrTiO_3$ or the like); a metal sulfide such as titanium sulfide, zinc sulfide, zirconium sulfide, copper sulfide, tin sulfide, indium sulfide, tungsten sulfide, cadmium sulfide, and silver sulfide; a metal selenide such as titanium selenide, zirconium selenide, indium selenide, and tungsten selenide; and a single semiconductor such as silicon and germanium, and it is preferable to use one or more of these semiconductors. In the present invention, it is preferable to use one or more selected from titanium oxide, zinc oxide, and tin oxide as the semiconductor.

The electron transport layer can be obtained by using a known film forming method depending on a material to be molded. Examples of a film forming method of the electron transport layer include, but are not limited to, a method of forming a film by applying a paste containing semiconductor fine particles onto a conductive substrate by a wet coating method such as a spin coating method, a doctor blade method, a squeegee method, or a screen printing method, and then removing a solvent or an additive by firing, a sputtering method, a vapor deposition method, an electrodeposition method, an electrocrystallization method, and a microwave irradiation method. An atmosphere for film forming is not particularly limited, and a film may be formed in the air.

A film thickness of the electron transport layer is not particularly limited, and is preferably about 10 nm to 500 nm, and more preferably about 50 nm to 400 nm, from a viewpoint of further collecting electrons from the photoelectric conversion layer. A particle diameter of the semiconductor is preferably 5 nm to 500 nm, and more preferably 10 nm to 100 nm.

In the present invention, a commercially available product may be used as the paste containing semiconductor fine particles, or a paste prepared by dispersing a commercially available semiconductor fine powder in a solvent may be used. Specific examples of the solvent used for preparing the paste include, but are not limited to, water; an alcohol solvent such as methanol, ethanol, and isopropyl alcohol; a ketone solvent such as acetone, methyl ethyl ketone, and methyl isobutyl ketone; and a hydrocarbon solvent such as n-hexane, cyclohexane, benzene, and toluene. These solvents may be used alone or as a mixed solvent of two or more thereof.

In the present invention, the semiconductor fine powder may be dispersed in the solvent after grinding the powder in a mortar or the like, or a disperser such as a ball mill, a paint conditioner, a vertical bead mill, a horizontal

bead mill, or an attritor may be used. At the time of preparing the paste, it is preferable to add a surfactant or the like in order to prevent aggregation of semiconductor fine particles, and it is preferable to add a thickener such as polyethylene glycol in order to increase a viscosity.

<Photoelectric Conversion Layer>

In the photoelectric conversion element according to the present invention, the photoelectric conversion layer 4 is preferably formed on the electron transport layer 3 as illustrated in FIG. 1, or the photoelectric conversion layer 9 is preferably formed on the electron transport layer 8 as illustrated in FIG. 2, but the present invention is not limited thereto.

In the present invention, in the case where the perovskite photoelectric conversion element is used, a perovskite material used as the photoelectric conversion layer represents a series of materials having a structure represented by a general formula$ABX_3$. Here, A, B, and X represent an organic cation or a monovalent metal cation, a metal cation, and a halide anion, respectively, and examples thereof include A = $K^+$, $Rb^+$, $Cs^+$, $CH_3NH_3^+$ (hereinafter, MA: methylammonium), $NH{=}CHNH_2^+$ (hereinafter, FA: formamidinium), and $CH_3CH_2NH_3^+$ (hereinafter, EA: ethylammonium); B = Pb and Sn; and X-$I^-$ and $Br^-$. Specifically, a layer containing a perovskite material represented by any composition such as $CH_3NH_3PbI_3$($MAPBI_3$), $C_2H_5NH_3PbI_3$, $CH_3NH_3PbBr_3$, $C_2H_5NH_3PbBr_3$, $CH_3NH_3PbBr_3$, $C_2H_5NH_3PbCl_3$, $CH_3NH_3PbCl_3$, $C_2H_5NH_3PbCl_3$, $CsSnI_3$, $CH_3NH_3SnI_3$, $C_2H_5NH_3SnI_3$, $CsSnBr_3$, $CH_3NH_3SnBr_3$, $C_2H_5NH_3SnBr_3$, $CsSnCl_3$, $CH_3NH_3SnCl_3$, and $C_2H_5NH_3SnCl_3$, or a perovskite material having a mixed cation and a mixed anion, represented by any composition such as $(FAMA)Pb(IBr)_3$, $K(FAMA)Pb(IBr)_3$, $Rb(FAMA)Pb(IBr)_3$, and $Cs(FAMA)Pb(IBr)_3$ can be used, but the present invention is not limited thereto. It is preferable to use one or more of these perovskite materials. A light absorbing agent other than the perovskite material may be contained.

The photoelectric conversion layer can be obtained by using a known film forming method such as an anti-solvent method or a two-stage method, depending on a material. In the present invention, it is preferable to form the photoelectric conversion layer by using the anti-solvent method, but the present invention is not limited thereto.

In the present invention, in the case of forming the photoelectric conversion layer by using the anti-solvent method, a perovskite precursor solution of a perovskite material is prepared, and a specific solvent is added during application of the perovskite precursor solution onto the electron transport layer by spin coating. After completion of the spin coating, heating is performed to obtain the photoelectric conversion layer.

A commercially available material may be used as a perovskite precursor, and in the present invention, it is preferable to use $PbI_2$/MAI (1:1)-DMF complex (Tokyo Chemical Industry Co., Ltd.) in the element configuration in FIG. 1, and it is preferable to use a precursor constituted by a lead halide, a methylammonium halide, a formamidine halide, and a cesium halide in any composition in the element configuration in FIG. 2, but the present invention is not limited thereto. MAI is an abbreviation for methylammonium iodide, and DMF is an abbreviation for N,N-dimethylformamide.

In the present invention, examples of a solvent of the perovskite precursor solution include, but are not limited to, dimethyl sulfoxide, dimethyl formamide, and $\gamma$-butyrolactone from a viewpoint of solubility of the precursor. These solvents may be used alone or in combination of two or more thereof, and it is preferable to use DMF or dimethyl sulfoxide.

The specific solvent added during the spin coating of the perovskite precursor solution is preferably a poor solvent, and examples thereof include, but are not limited to, an aromatic organic solvent such as toluene, benzene, chlorobenzene, orthodichlorobenzene, and nitrobenzene; an ether solvent such as diethyl ether and tetrahydrofuran (THF); an alcohol solvent such as methanol, ethanol, isopropanol, butanol, and octanol; a long-chain hydrocarbon solvent such as hexane; pyridine; and acetonitrile. These solvents may be used alone or in combination of two or more thereof. Among these, an aromatic organic solvent is preferable, and chlorobenzene and toluene are more preferable. An addition amount is not particularly limited as long as the specific solvent can sufficiently diffuse into an entire element substrate formed by the conductive support, the hole blocking layer, and the electron transport layer, which are rotated with the spin coating.

In the present invention, a temperature at which the photoelectric conversion layer (perovskite layer) is heated with a hot plate or the like is preferably 50°C to 150°C, and more preferably 70°C to 120°C, from a viewpoint of generating the perovskite material from the precursor. A heating time is preferably about 10 minutes to 90 minutes, and more preferably about 30 minutes to 60 minutes.

An atmosphere during film formation of the perovskite material is preferably a dry atmosphere, and more preferably a dry inert gas atmosphere such as a glove box, from a viewpoint that a perovskite solar cell can be produced with good reproducibility and high efficiency by preventing incorporation of moisture. It is preferable to perform dehydration with a molecular sieve or the like to use a solvent having a low moisture content.

A thickness of the photoelectric conversion layer (perovskite layer) in the present invention is preferably 50 nm to 1000 nm, and more preferably 200 nm to 700 nm, from a viewpoint of further preventing performance degradation due to defects and peeling.

<Hole Transport Layer>

The hole transport layer is a layer having a function of transporting holes. The hole transport layer may include, for example, a conductor, a semiconductor, and an organic hole transport material. The material can function as a hole transport material that receives holes from the photoelectric conversion layer (perovskite layer) and transports the holes. The present embodiment can achieve a high efficiency even if a dopant as an additive is not contained, but the dopant may be contained for a purpose of further improving a hole transport characteristic. The dopant may reduce durability of the photoelectric conversion element and may shorten a life of the entire element, and thus it is desirable to reduce the amount of the dopant to be used.

The hole transport layer in the present invention is a layer containing the compound represented by the general formula (1) as the hole transport material for a photoelectric conversion element. Since the compound represented by the general formula (1) is used in the hole transport layer, a photoelectric conversion element and a solar cell with high durability can be formed. When the compound represented by the general formula (1) is used in the hole transport layer, a good photoelectric conversion efficiency can be achieved. When the compound represented by the general formula (1) is used in the hole transport layer, a sufficient photoelectric conversion efficiency can be achieved, and a current can be efficiently extracted even if the dopant was reduced or not used. By reducing or not using the dopant, the photoelectric conversion element and the solar cell can be formed with a simple process and at a low cost. In the hole transport layer in the present invention, the compound represented by the general formula (1) may be used alone or in combination of two or more thereof, or may be used in combination with other hole transport materials and the like which do not belong to the present invention. Specific examples of the other hole transport materials include a compound semiconductor containing monovalent copper such as CuI, $CuInSe_2$, and CuS; and a compound containing a metal other than copper such as GaP, NiO, CoO, FeO, $Bi_2O_3$, MoOz, and $Cr_2O_3$, and examples of an organic hole transport material include a polythiophene derivative such as poly-3-hexylthiophene (P3HT) and polyethylene dioxythiophene (PEDOT); a fluorene derivative such as 2,2',7,7'-tetrakis(N,N-di-p-methoxyphenylamine)-9,9'-spirobifluorene (Spiro-OMeTAD); a carbazole derivative such as polyvinyl carbazole; a triphenylamine derivative such as poly[bis(4-phenyl)(2,4,6-trimethylphenyl)amine] (PTAA); a diphenylamine derivative; a polysilane derivative; and a polyaniline derivative. The hole transport layer may contain a dopant (oxidizing agent) as an additive as necessary.

The hole transport layer 5 illustrated in FIG. 1 in the present invention is preferably formed on the photoelectric conversion layer 4 (perovskite layer) by coating and drying, and the hole transport layer 10 illustrated in FIG. 2 is preferably formed on the photoelectric conversion layer 9 (perovskite layer) by coating and drying. Specific examples of a film forming method include spin coating, screen printing, roll coating, dip coating, spraying, knife coating, bar coating, die coating, and curtain coating, and in the present invention, a spin coating method is preferable. Conditions of the spin coating can be appropriately set.

In the present invention, examples of a solvent used for film formation include, but are not limited to, an aromatic organic solvent such as benzene, toluene, xylene, mesitylene, tetralin(1,2,3,4-tetrahydronaphthalene), monochlorobenzene, o-dichlorobenzene, m-dichlorobenzene, p-dichlorobenzene, and nitrobenzene; a halogenated alkyl organic solvent such as dichloromethane, chloroform, 1,2-dichloroethane, 1,1,2-trichloroethane, and dichloromethane; a nitrile solvent such as benzonitrile and acetonitrile; an ether solvent such as tetrahydrofuran, dioxane, diisopropyl ether, c-pentyl methyl ether, ethylene glycol dimethyl ether, ethylene glycol diethyl ether, and propylene glycol monomethyl ether; an ester solvent such as ethyl acetate and propylene glycol monomethyl ether acetate; and an alcohol solvent such as methanol, isopropanol, n-butanol, propylene glycol, 1,3-butanediol, 1,4-butanediol, 2,3-butanediol, cyclohexanol, and 2-n-butoxyethanol. The solvent may be used alone or in combination of two or more thereof, and a solvent to be used may be selected depending on a structure. In particular, it is preferable to use an aromatic organic solvent and a halogenated alkyl organic solvent.

In the present invention, conditions for drying the hole transport layer after coating as described above are not particularly limited, and the drying is preferably performed to an extent that the solvent can be removed, and heating is preferably performed at about 50°C to 120°C with the hot plate or the like, and a heating time is preferably about 10 minutes to 60 minutes.

In the present invention, a film thickness of the hole transport layer is preferably about 10 nm to 500 nm from a viewpoint of further improving the photoelectric conversion efficiency.

An atmosphere during film formation of the hole transport layer is preferably a dry atmosphere, and more preferably a dry atmosphere such as a glove box, from a viewpoint that a perovskite solar cell can be produced with good reproducibility and high efficiency by preventing incorporation of moisture. It is preferable to perform dehydration with a molecular sieve or the like to use a solvent having a low moisture content.

<Additive>

In the present invention, a composition containing the hole transport material for a photoelectric conversion

element, and an additive such as a dopant (or an oxidizing agent) and a basic compound (or a basic additive), may be used in the hole transport layer. Adding an additive in the hole transport layer to improve a carrier concentration of the hole transport material for a photoelectric conversion element in the hole transport layer (i.e., doping) leads to an improvement in the conversion efficiency of the photoelectric conversion element. In the present invention, in the case where a composition containing a dopant and a basic additive as additives is used in the hole transport layer, the composition preferably contains 0 equivalents to 3.5 equivalents of the additive with respect to 1 equivalent of the hole transport material for a photoelectric conversion element.

In the present invention, in the case where a dopant is contained for the purpose of further improving the hole transport characteristic, specific examples of the dopant include tris(2-(1H-pyrazol-1-yl)-4-tert-butylpyridine)cobalt(III)tri[bis(trifluoromethane)sulfonimide] (FK209), bis(trifluoromethylsulfonyl)imide lithium (LiTFSI), bis(trifluoromethanesulfonyl)imide silver, $NOSbF_6$, SbCls, and $SbF_5$. In the present invention, it is preferable to use bis(trifluoromethylsulfonyl)imide lithium (LiTFSI).

In the present invention, in the case where a composition containing a dopant is used, the composition preferably contains 0 equivalents to 2.0 equivalents of the dopant, and more preferably contains 0 equivalents to 0.5 equivalents of the dopant with respect to 1 equivalent of the hole transport material for a photoelectric conversion element.

A basic compound (basic additive) may be contained as an additive for the hole transport layer. In the present invention, in the case where a basic compound is contained, specific examples thereof include 4-tert-butylpyridine (TBP), 2-picoline, and 2,6-lutidine. The basic compound is often used in combination when a dopant is used. Also in the present invention, when a dopant is used, it is desirable to use a basic compound in combination.

In the present invention, in the case where a composition containing a basic compound is used, the composition preferably contains 0 equivalents to 5 equivalents of the basic compound, and more preferably contains 0 equivalents to 3 equivalents of the basic compound with respect to 1 equivalent of the hole transport material for a photoelectric conversion element.

<Counter Electrode>

In the present invention, the counter electrode 6 illustrated in FIG. 1 is disposed to face the conductive support 1 and is formed on the hole transport layer 5, and in the element configuration illustrated in FIG. 2, the counter electrode 11 is similarly formed on the hole transport layer 10, so that charges can be exchanged with the hole transport layer. In the photoelectric conversion element according to the present invention, a metal electrode is preferably provided as a counter electrode on the hole transport layer, and an electron blocking layer may be added between the hole transport layer and the counter electrode.

Specific examples of a material used for the counter electrode include a metal such as platinum, titanium, stainless steel, aluminum, gold, silver, and nickel, and an alloy thereof.

In the present invention, the counter electrode is preferably a material that can be formed by using a method such as vapor deposition. A film thickness of the counter electrode is not particularly limited, and is preferably, for example, about 50 nm to 150 nm.

In the photoelectric conversion element according to the present invention, the conductive support serves as an anode, and the counter electrode serves as a cathode. Light such as sunlight is preferably emitted from a conductive support side. Upon irradiation with sunlight or the like, the photoelectric conversion layer (perovskite layer) absorbs light and becomes an excited state, and electrons and holes are generated. When the electrons move to the electrode through the electron transport layer, and the holes move to the electrode through the hole transport layer, a current flows, and a function as a photoelectric conversion element is achieved.

When a performance (a characteristic) of the photoelectric conversion element according to the present invention is evaluated, a short-circuit current density, an open-circuit voltage, a fill factor, and a photoelectric conversion efficiency are measured. The short-circuit current density represents a current per 1 $cm^2$ flowing between both terminals when an output terminal is short-circuited, and the open-circuit voltage represents a voltage between both terminals when the output terminal is opened. The fill factor is a value obtained by dividing a maximum output (product of a current and a voltage) by a product of the short-circuit current density and the open-circuit voltage, and is mainly influenced by an internal resistance. The photoelectric conversion efficiency is obtained as a value expressed in percentage by multiplying, by 100, a value obtained by dividing the maximum output (W) by a light intensity (W) per 1 $cm^2$.

In the present invention, when durability of the photoelectric conversion element is evaluated, the evaluation is performed with a retention rate (%) of the photoelectric conversion efficiency over time in a dry atmosphere. The dry atmosphere is a state in which a drying agent such as silica gel is placed in a sealed container such as a desiccator and a humidity is 5% RH or less.

The photoelectric conversion element according to the present invention can be applied to a solar cell, various optical sensors, and the like. The solar cell according to the present invention is preferably a perovskite solar cell, and the perovskite solar cell is obtained by forming, as a cell, a photoelectric conversion element including a hole transport

layer for a photoelectric conversion element using a compound represented by the general formula (1) as a hole transport material for a photoelectric conversion element, arranging a required number of the cells into a module, and providing a predetermined electric wiring.

Although a preferred embodiment has been described above, the present invention is not limited to the embodiment, and may be appropriately modified without departing from the scope of the present invention.

## EXAMPLES

Hereinafter, the present invention will be described in detail with Examples with reference to the drawings, but the present invention is not limited to the following Examples. Compounds obtained in Synthesis Examples were identified by $^{1}$H-NMR (1H-NMR (nuclear magnetic resonance apparatus manufactured by JEOL Ltd., JNM-ECZ400S/L1 type)).

[Synthesis Example 1] Synthesis of Compound (A-1)

To a reaction vessel was added 3,3'-dibromo-5,5'-bis(trimethylsilyl)-2,2'-bithiophene (10.0 g, manufactured by TCI), and argon substitution was performed. Dehydrated tetrahydrofuran (106 mL) was added, and the mixture was cooled to -78°C with a dry ice-acetone bath. A hexane solution of n-butyllithium (1.55 mol/L, 29.0 mL, manufactured by Kanto Chemical Co., Inc.) was added thereto dropwise. Thereafter, the temperature was raised to -30°C over 4 hours, and dimethylcarbamoyl chloride (1.2 mL, manufactured by TCI) was added thereto. After stirring at -30°C for 20 minutes, the temperature was gradually raised to 0°C over 3 hours. Thereafter, the temperature was raised to room temperature. Water (150 mL) was added, liquid separation was performed, and the organic layer was washed with water (150 mL) and saturated brine (100 mL). The aqueous layer was extracted with chloroform (40 mL) three times. The organic layer was dried with anhydrous magnesium sulfate, and a solvent was distilled off to obtain a red solid as a crude product. The crude product was purified with a silica gel column (hexane/ethyl acetate = 97/3 (volume ratio)) to obtain a compound represented by the following formula (9) as a red solid (yield: 5.76 g, yield rate: 80%).

A reaction vessel was charged with the compound represented by the formula (9) obtained above (3.5 g) and tetrahydrofuran (hereinafter, referred to as THF, 21 mL), and a mixture was cooled to 5°C or lower in an ice bath. N-bromosuccinimide (4.9 g, manufactured by TCI) and N,N-dimethylformamide (hereinafter, referred to as DMF, 7 mL) were added thereto in several times. Thereafter, the temperature was returned to room temperature, and the mixture was stirred for 2 hours. After completion of a reaction, the reaction solution was added to a beaker containing water (400 mL). After stirring for 30 minutes, the solution was filtered. The obtained solid was dried at 70°C under reduced pressure to obtain a compound represented by the following formula (10) as a purple solid (yield: 3.53 g, yield rate: 97%).

[Chem. 19]

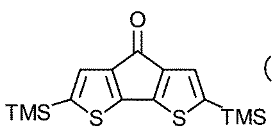
(9)

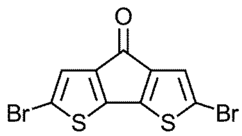
(10)

To a reaction vessel were added 4-bromoanisole (10.60 g, manufactured by TCI), diphenylamine (8.72 g, manufactured by TCI), palladium acetate (0.015 g), sodium-t-butoxide (11.90 g, manufactured by Kanto Chemical Co., Inc.), tri-t-butylphosphine (0.017 g, manufactured by Kanto Chemical Co., Inc.), and toluene (110 mL), and deaeration was performed under reduced pressure. A mixture was heated to reflux for 3 hours in an argon atmosphere. After completion of a reaction, the reaction mixture was cooled and suction-filtered. The residue was washed with toluene (50 mL), and the filtrate was concentrated. The obtained crude product was purified with an amino silica gel column (toluene/hexane = 1/1 (volume ratio)), and then recrystallized (toluene/methanol) to obtain a compound represented by the following formula (11) as a white solid (yield: 11.02 g, yield rate: 78%).

The compound represented by the formula (11) obtained above (10.50 g) and THF (71 mL) were added to a reaction vessel, and the mixture was cooled to 5°C or lower. N-bromosuccinimide (14.30 g, manufactured by TCI) was added thereto, and the mixture was stirred at 5°C or lower for 2 hours. After completion of a reaction, the reaction solution was poured into a beaker containing 300 mL of water. Toluene (100 mL) was added thereto. The resultant solution was separated, and the aqueous layer was extracted twice with toluene (40 mL). The organic layer was dried with sodium sulfate, and concentrated. The crude product was purified with a silica gel column (toluene/hexane = 1:1 (volume ratio)) to obtain a compound represented by the following formula (12) as a yellow oily matter (yield: 16.50 g, yield rate: 100%).

[Chem. 20]

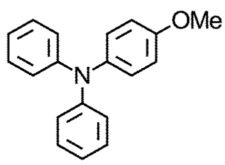
(11)
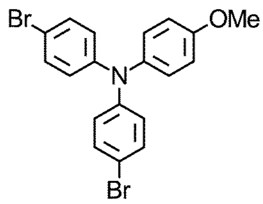
(12)

The compound represented by the formula (12) obtained above (16.40 g), 4,4'-dimethoxydiphenylamine (2.90 g, manufactured by TCI), tris(dibenzylideneacetone)dipalladium (0) (0.68 g, manufactured by Kanto Chemical Co., Inc.), sodium-t-butoxide (3.67 g, manufactured by Kanto Chemical Co., Inc.), 1,1'-bis(diphenylphosphino)ferrocene (0.56 g, manufactured by Kanto Chemical Co., Inc.), and toluene (25 mL) were added to a reaction vessel, and deaeration was performed under reduced pressure. The mixture was heated to reflux for 4 hours in an argon atmosphere. After completion of a reaction, the reaction mixture was cooled to 40°C or lower and suction-filtered. The residue was washed with toluene (50 mL), and the filtrate was concentrated. The crude product was purified with a silica gel column (toluene/hexane = 1/1 (volume ratio)) to obtain a compound represented by the following formula (13) as a yellow solid (yield: 2.76 g, yield rate: 38%).

The compound represented by the formula (13) obtained above (10.0 g) and anhydrous THF (130 mL) were added to a reaction vessel, and deaeration was performed under reduced pressure. The mixture was cooled to -70°C or lower with an acetone-dry ice bath in an argon atmosphere. An n-butyllithium hexane solution (1.55 M, 17.0 mL, manufactured by Kanto Chemical Co., Inc.) was added dropwise thereto little by little. After completion of the dropwise addition, the mixture was stirred at -70°C or lower for 1 hour, and triisopropyl borate (6.0 mL, manufactured by TCI) was added dropwise. After completion of the dropwise addition, the temperature was gradually raised to room temperature, and the mixture was stirred at room temperature for 1 hour. Water (55 mL) was poured into the reaction solution to terminate the reaction. The reaction solution was separated, and the aqueous layer was extracted twice with toluene (40 mL). The organic layer was dried with sodium sulfate, and concentrated. The crude product was purified with a silica gel column (toluene and then THF) to obtain a compound represented by the following formula (14) as a yellow powder (yield: 5.75 g, yield rate: 62%).

[Chem. 21]

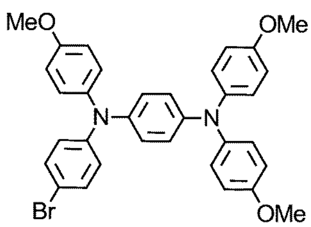
(13)
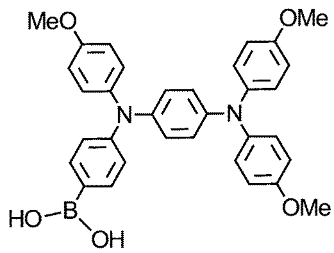
(14)

The compound represented by the formula (10) (1.00 g), the compound represented by the formula (14) (4.59 g), potassium carbonate (19.0 g, manufactured by Kanto Chemical Co., Inc.), tetrakis(triphenylphorin)palladium (0) (0.15 mg, manufactured by Kanto Chemical Co., Inc.), and toluene (150 mL) were added to a reaction vessel, and deaeration was performed. Thereafter, the mixture was heated to reflux in an argon atmosphere for 68 hours. After completion of a reaction, the reaction mixture was cooled to 50°C or lower and filtered through Celite. The residue was washed with toluene (100 mL), and the filtrate was concentrated. The crude product was purified with a silica gel column (toluene and then toluene/acetone = 100/1 (volume ratio)). The product was purified again with a silica gel column (toluene/acetone = 100/1 (volume ratio)) to obtain a compound represented by the following formula (15) as a black green powder (yield: 1.34 g, yield rate: 40%).

[Chem. 22]

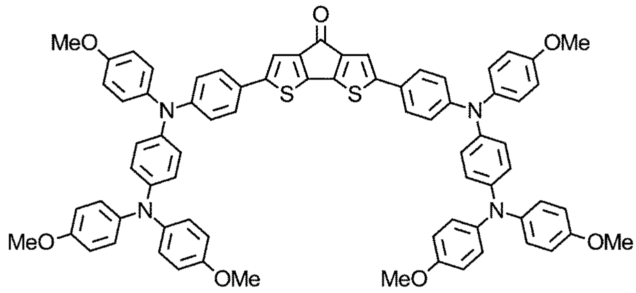

(15)

The compound represented by the formula (15) (0.57 g), malononitrile (0.13 g, manufactured by TCI), and THF (15 mL) were added to a reaction vessel and stirred in an argon atmosphere. A solution of acetic acid-pyridine (volume ratio 1:1) (3.6 mL) was added thereto. The mixture was stirred under heating and refluxing for 6 hours. After completion of a reaction, water (30 mL) and toluene (20 mL) were added thereto. The reaction solution was separated, and the aqueous layer was extracted twice with toluene (30 mL). The organic layer was dried with magnesium sulfate, and concentrated. The crude product was purified with a column (toluene) to obtain a compound represented by the following formula (A-1) as a black green powder (yield: 0.493 g, yield rate: 83%).

$^1$H-NMR (400 MHz, DMSO-$d_6$): δ (ppm) = 3.72 (12H), 3.73 (6H), 6.7 to 6.8 (8H), 6.89 (8H), 6.92 (8H), 7.00 (8H), 7.05 (4H), 7.23 (2H), 7.33 (4H).

[Chem. 23]

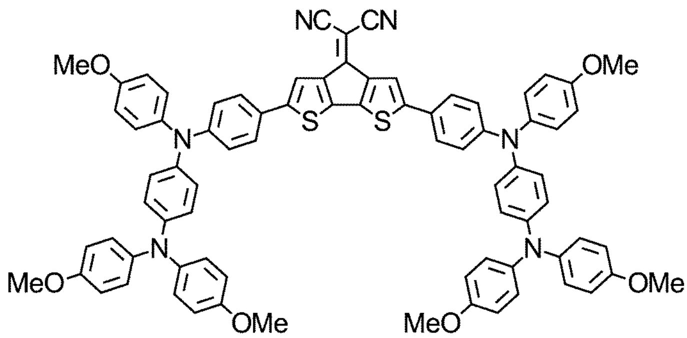

(A−1)

[Synthesis Example 2] Synthesis of Compound (A-11)

To a reaction vessel were added 3,6-dibromocarbazole (5.0 g, manufactured by TCI), 1-chloro-4-fluorobenzene (7.83 g, manufactured by TCI), cesium carbonate (19.5 g, manufactured by Kanto Chemical Co., Inc.), and DMF (15 mL). The mixture was stirred at 130°C for 38 hours in an argon atmosphere. After completion of a reaction, the reaction solution was poured into a beaker containing saturated brine (250 mL). The mixture solution was filtered, and the residue was washed with water (50 mL) and methanol (10 mL). The crude product was purified with a silica gel column (toluene) to obtain a compound represented by the following formula (16) as a white powder (yield: 6.04 g, yield rate: 92%).

[Chem. 24]

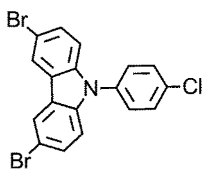

(16)

The compound represented by the formula (16) (5.0 g), 4,4'-dimethoxydiphenylamine (5.27 g, manufactured by TCI), palladium acetate (0.11 g, manufactured by Kanto Chemical Co., Inc.), sodium-t-butoxide (5.19 g, manufactured

by Kanto Chemical Co., Inc.), tri-t-butylphosphine (33% xylene solution) (0.67 g, manufactured by Kanto Chemical Co., Inc.), and toluene (30 mL) were added to a reaction vessel, and deaeration was performed under reduced pressure in an argon atmosphere. The mixture was heated to reflux for 4 hours, and after completion of a reaction, the reaction solution was filtered through Celite. The residue was washed with toluene (30 mL), and the filtrate was concentrated. The crude product was purified with a silica gel column (toluene and then toluene/acetone = 100/1 (volume ratio)) to obtain a compound represented by the following formula (17) as a yellow powder (yield: 3.10 g, yield rate: 39%).

The compound represented by the formula (17) obtained above (3.10 g), bispinacolate diboron (2.13 g, manufactured by TCI), potassium acetate (2.06 g, manufactured by Kanto Chemical Co., Inc.), 1,1'bis(diphenylphosphino)ferrocene dichloropalladium (0.343 g, manufactured by Kanto Chemical Co., Inc.), and 1,4-dioxane (27 mL) were added to a reaction vessel, and deaeration was performed under reduced pressure in an argon atmosphere. The mixture was heated to reflux for 37 hours, and after completion of a reaction, the reaction solution was cooled to 40°C or lower and filtered through Celite. The filtrate was concentrated, and the crude product was purified with a silica gel column (toluene/acetone = 200/1 (volume ratio)). The product was purified again with a silica gel column (toluene) to obtain a compound represented by the following formula (18) as a yellow powder (yield: 2.56 g, yield rate: 74%).

[Chem. 25]

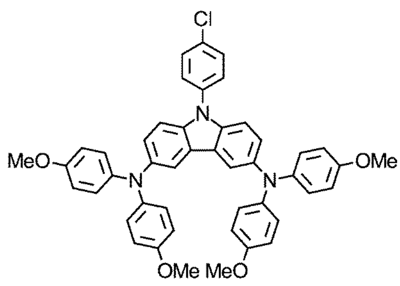
(17)

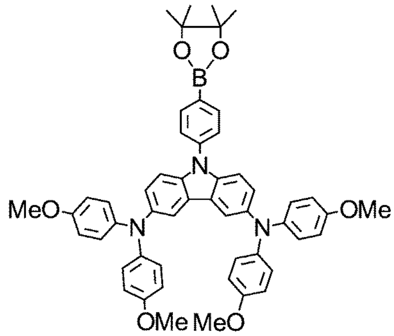
(18)

The compound represented by the formula (10) (0.43 g), the compound represented by the formula (18) (2.56 g), tetrakis(triphenylphosphine)palladium (0) (0.058 g, manufactured by Kanto Chemical Co., Inc.), potassium carbonate (0.44 g, manufactured by Kanto Chemical Co., Inc.), toluene (7 mL), ethanol (2 mL), and water (2 mL) were added to a reaction vessel, and deaeration was performed under reduced pressure. The mixture was heated to reflux for 7 hours in an argon atmosphere. After completion of a reaction, toluene (20 mL) and water (10 mL) were added thereto. The reaction solution was separated, the aqueous layer was extracted twice with toluene (20 mL), and the organic layer was concentrated. The crude product was purified with a silica gel column (toluene/acetone = 100/1 (volume ratio)) to obtain a compound represented by the following formula (19) as a black blue powder (yield: 1.65 g, yield rate: 84%).

[Chem. 26]

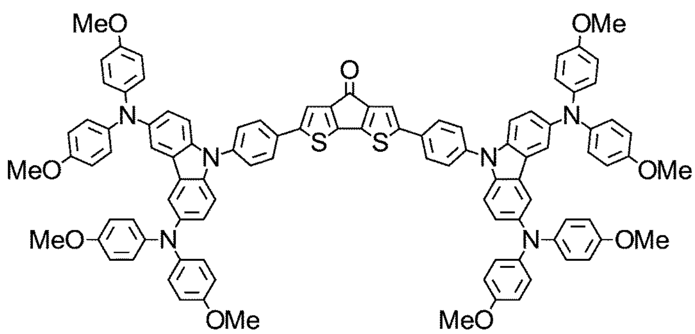
(19)

The compound represented by the formula (19) (0.56 g), malononitrile (0.09 g, manufactured by TCI), and THF (15 mL) were added to a reaction vessel and stirred in an argon atmosphere. A solution of acetic acid-pyridine (1:1) (2.6 mL) was added thereto. The mixture was stirred under heating and refluxing for 16 hours. After completion of a reaction, water (30 mL) was added thereto. The mixture solution was filtered, and the residue was washed with water (30 mL) and methanol (20 mL). The crude product was recrystallized (chloroform/acetone) to obtain a compound represented

by the following formula (A-11) as a green powder (yield: 0.52 g, yield rate: 91%).

$^1$H-NMR (400 MHz, THF-ds): δ (ppm) = 3.68 (24H), 6.72 (16H), 6.91 (16H), 7.08 (4H), 7.3 (4H), 7.66 (8H), 7.76 (2H), 7.90 (4H).

[Chem. 27]

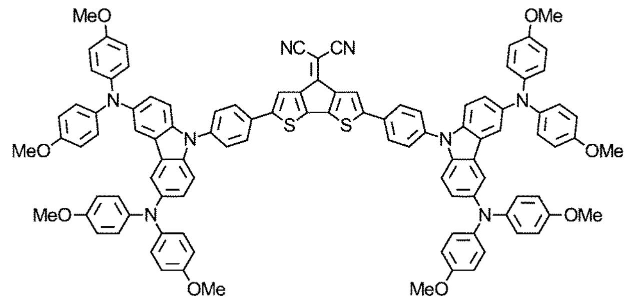

(A－11)

[Synthesis Example 3] Synthesis of Compound (A-12)

The compound represented by the formula (10) (0.43 g), a compound represented by the following formula (20) (2.04 g), tetrakis(triphenylphosphine)palladium (0) (0.058 g, manufactured by Kanto Chemical Co., Inc.), potassium carbonate (0.44 g, manufactured by Kanto Chemical Co., Inc.), toluene (7 mL), ethanol (2 mL), and water (2 mL) were added to a reaction vessel, and deaeration was performed under reduced pressure. The mixture was heated to reflux for 7 hours in an argon atmosphere. After completion of a reaction, toluene (20 mL) and water (10 mL) were added thereto. The reaction solution was separated, the aqueous layer was extracted twice with toluene (20 mL), and the organic layer was concentrated. The crude product was purified with a silica gel column (toluene) and a silica gel column (toluene:hexane = 2/1 (volume ratio)) to obtain a compound represented by the following formula (21) as a black purple powder (yield: 1.42 g, yield rate: 86%).

[Chem. 28]

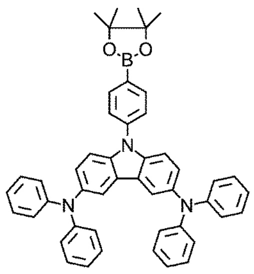

(20)

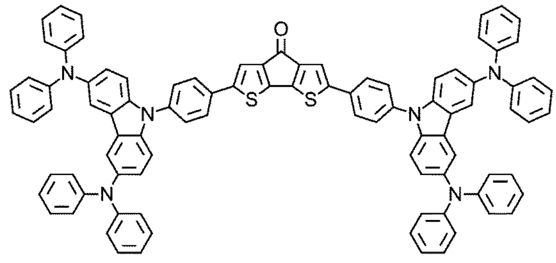

(21)

The compound represented by the formula (21) (0.60 g), malononitrile (0.12 g, manufactured by TCI), and THF (19 mL) were added to a reaction vessel and stirred in an argon atmosphere. A solution of acetic acid-pyridine (1:1) (3.2 mL) was added thereto. The mixture was stirred under heating and refluxing for 54 hours. After completion of a reaction, the reaction solution was poured into water (200 mL). The mixture solution was filtered, and the residue was washed with water (30 mL) and methanol (20 mL). The crude product was washed with toluene and recrystallized (chloroform/acetone) to obtain a compound represented by the following formula (A-12) as a green powder (yield: 0.30 g, yield rate: 48%).

$^1$H-NMR (400 MHz, THF-ds): δ (ppm) = 6.84 (8H), 7.00 (16H), 7.13 (16H), 7.19 (4H), 7.39 (4H), 7.55 (2H), 7.68 (4H), 7.86 (4H), 7.93 (4H).

[Chem. 29]

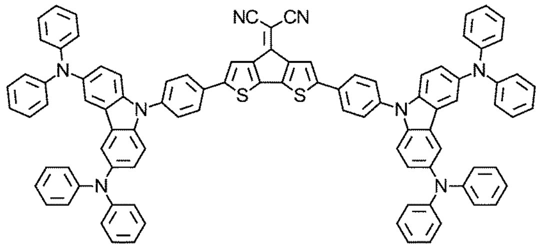

(A-12)

[Synthesis Example 4] Synthesis of Compound (A-26)

The compound represented by the formula (10) (0.43 g), a compound represented by the following formula (22) (2.52 g), tetrakis(triphenylphosphine)palladium (0) (0.058 g, manufactured by Kanto Chemical Co., Inc.), potassium carbonate (0.44 g, manufactured by Kanto Chemical Co., Inc.), toluene (7 mL), ethanol (2 mL), and water (2 mL) were added to a reaction vessel, and deaeration was performed under reduced pressure. The mixture was heated to reflux for 7 hours in an argon atmosphere. After completion of a reaction, toluene (20 mL) and water (10 mL) were added thereto. The reaction solution was separated, the aqueous layer was extracted twice with toluene (20 mL), and the organic layer was concentrated. The crude product was purified with a silica gel column (toluene/ethyl acetate = 50/1 (volume ratio)) to obtain a compound represented by the following formula (23) as a black powder (yield: 1.52 g, yield rate: 75%).

[Chem. 30]

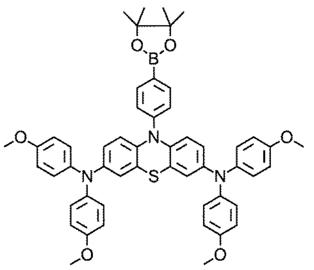

(22)

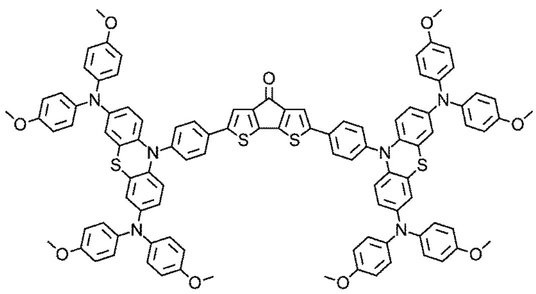

(23)

The compound represented by the formula (23) (0.56 g), malononitrile (0.09 g, manufactured by TCI), and THF (15 mL) were added to a reaction vessel and stirred in an argon atmosphere. A solution of acetic acid-pyridine (1:1) (2.6 mL) was added thereto. The mixture was stirred under heating and refluxing for 16 hours. After completion of a reaction, water (30 mL) was added thereto. The mixture solution was filtered, and the residue was washed with water (30 mL) and methanol (20 mL). The crude product was recrystallized (chloroform/acetone) to obtain a compound represented by the following formula (A-26) as a green powder (yield: 0.51 g, yield rate: 88%).

$^1$H-NMR (400 MHz, THF-ds): $\delta$ (ppm) = 3.67 (24H), 6.65 (16H), 6.84 (16H), 6.96 (4H), 7.11 (4H), 7.57 (8H), 7.59 (2H), 7.80 (4H).

[Chem. 31]

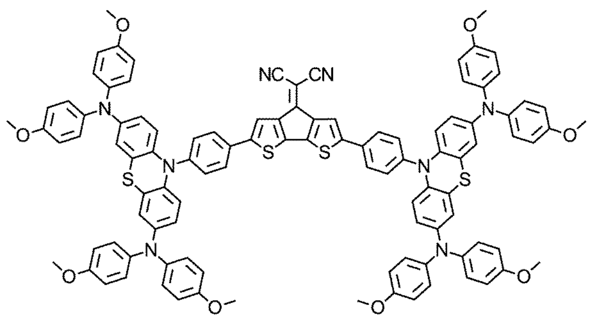

(A−26)

[Synthesis Example 5] Synthesis of Compound (A-41)

The compound represented by the formula (10) (0.43 g), a compound represented by the following formula (24) (2.48 g), tetrakis(triphenylphosphine)palladium (0) (0.058 g, manufactured by Kanto Chemical Co., Inc.), potassium carbonate (0.44 g, manufactured by Kanto Chemical Co., Inc.), toluene (7 mL), ethanol (2 mL), and water (2 mL) were added to a reaction vessel, and deaeration was performed under reduced pressure. The mixture was heated to reflux for 7 hours in an argon atmosphere. After completion of a reaction, toluene (20 mL) and water (10 mL) were added thereto. The reaction solution was separated, the aqueous layer was extracted twice with toluene (20 mL), and the organic layer was concentrated. The crude product was purified with a silica gel column (toluene/ethyl acetate = 50/1 (volume ratio)) to obtain a compound represented by the following formula (25) as a black powder (yield: 1.52 g, yield rate: 77%).

[Chem. 32]

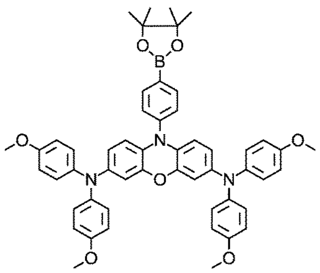

(24)

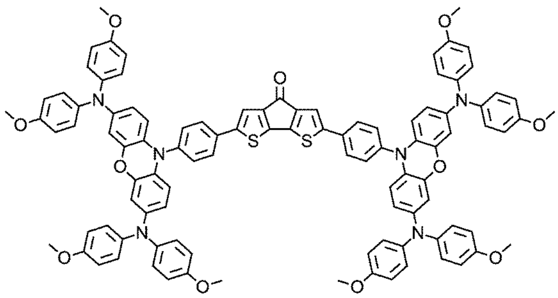

(25)

The compound represented by the formula (25) (0.55 g), malononitrile (0.09 g, manufactured by TCI), and THF (15 mL) were added to a reaction vessel and stirred in an argon atmosphere. A solution of acetic acid-pyridine (1:1) (2.6 mL) was added thereto. The mixture was stirred under heating and refluxing for 16 hours. After completion of a reaction, water (30 mL) was added thereto. The mixture solution was filtered, and the residue was washed with water (30 mL) and methanol (20 mL). The crude product was recrystallized (chloroform/acetone) to obtain a compound represented by the following formula (A-41) as a green powder (yield: 051. g, yield rate: 90%).

$^1$H-NMR (400 MHz, THF-ds): $\delta$ (ppm) = 3.68 (24H), 6.68 (16H), 6.88 (16H), 7.00 (4H), 7.15 (4H), 7.62 (8H), 7.64 (2H), 7.85 (4H).

[Chem. 33]

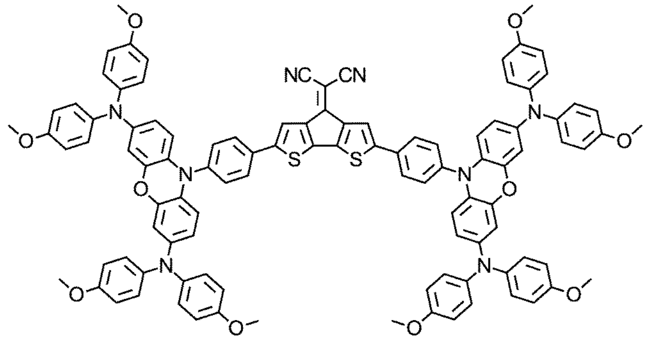

(A−41)

[Synthesis Example 6] Synthesis of Compound (A-56)

The compound represented by the formula (10) (0.88 g), [4-[bis(4-methoxyphenyl)amino]phenyl]boronic acid (2.10 g, manufactured by TCI), tetrakis(triphenylphosphine)palladium (0) (0.12 g, manufactured by Kanto Chemical Co., Inc.), potassium carbonate (0.90 g, manufactured by Kanto Chemical Co., Inc.), toluene (13 mL), ethanol (4.5 mL), and water (4.5 mL) were added to a reaction vessel, and deaeration was performed under reduced pressure. The mixture was heated to reflux for 6 hours in an argon atmosphere. After completion of a reaction, toluene (10 mL) and water (10 mL) were added thereto. The reaction solution was separated, and the aqueous layer was extracted twice with toluene (20 mL). The organic layer was concentrated, and the crude product was purified with a silica gel column (toluene/acetone = 100/1 (volume ratio)). The product was purified again with a silica gel column (toluene) to obtain a compound represented by the following formula (26) as a black green powder (yield: 1.86 g, yield rate: 93%).

[Chem. 34]

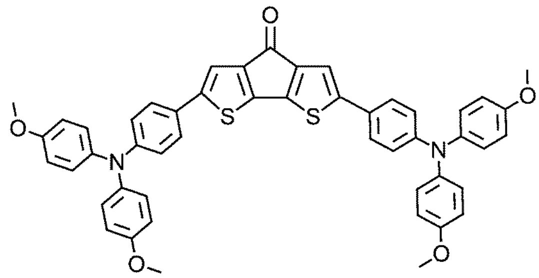

(26)

The compound represented by the formula (26) (0.6 g), diethyl malonate (0.37 g, manufactured by TCI), and THF (14 mL) were added to a reaction vessel and stirred in an argon atmosphere. After cooling to 5°C or lower in an ice bath, carbon tetrachloride (0.9 mL, manufactured by Junsei Chemical Co., Ltd.), titanium tetrachloride (0.5 mL, manufactured by Wako Pure Chemical Industries, Ltd.), and THF (8 mL) were added thereto. After cooling to 5°C or lower again, pyridine (1 mL) and THF (1 mL) were further added thereto. Thereafter, the temperature was raised to room temperature, and stirring was performed at room temperature for 17 hours. After completion of a reaction, the reaction solution was poured into a beaker containing 50 mL of water, and toluene (30 mL) was added thereto. The reaction solution was separated, and the organic layer was washed twice with a 10% aqueous sodium carbonate solution (50 mL). The organic layer was dried with magnesium sulfate, and concentrated. The crude was purified with a silica gel column (toluene) to obtain a compound represented by the following formula (A-56) as a black green powder (yield: 0.56 g, yield rate: 79%).

$^1$H-NMR (400 MHz, $CDCl_3$): δ (ppm) = 1.38 (6H), 3.81 (12H), 4.40 (4H), 6.84 (8H), 6.90 (4H), 7.07 (8H), 7.31 (4H), 7.36 (2H).

[Chem. 35]

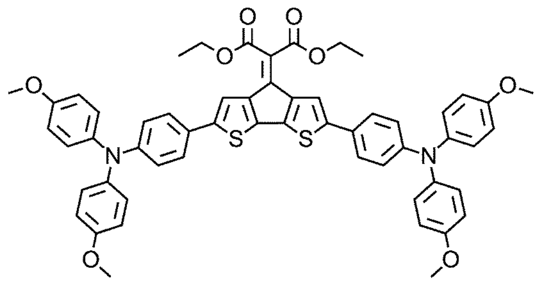

(A−56)

[Synthesis Example 7] Synthesis of Compound (A-58)

A compound represented by the following formula (27) (1.33 g), [4-[bis(4-methoxyphenyl)amino]phenyl]boronic acid (2.10 g, manufactured by TCI), tetrakis(triphenylphosphine)palladium (0) (0.12 g, manufactured by Kanto Chemical Co., Inc.), potassium carbonate (0.90 g, manufactured by Kanto Chemical Co., Inc.), toluene (13 mL), ethanol (4.5 mL), and water (4.5 mL) were added to a reaction vessel, and deaeration was performed under reduced pressure. The mixture was heated to reflux for 6 hours in an argon atmosphere. After completion of a reaction, toluene (10 mL) and water (10 mL) were added thereto. The reaction solution was separated, and the aqueous layer was extracted twice with toluene (20 mL). The organic layer was concentrated, and the crude product was purified with a silica gel column (toluene) to obtain a compound represented by the following formula (A-58) as a black green powder (yield: 2.11 g, yield rate: 86%).

$^1$H-NMR (400 MHz, $CDCl_3$): δ (ppm) = 1.32 (6H), 3.81 (12H), 4.59 (4H), 6.84 (8H), 6.90 (4H), 7.08 (8H), 7.33 (4H), 7.69 (2H).

[Chem. 36]

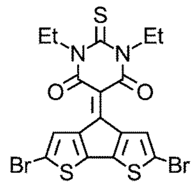

(27)

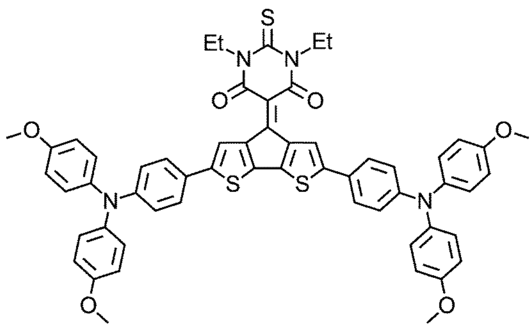

(A−58)

[Synthesis Example 8] Synthesis of Compound (A-63)

The compound represented by the formula (26) (0.6 g), methyl cyanoacetate (0.23 g, manufactured by TCI), and THF (14 mL) were added to a reaction vessel and stirred in an argon atmosphere. After cooling to 5°C or lower in an ice bath, carbon tetrachloride (0.9 mL, manufactured by Junsei Chemical Co., Ltd.), titanium tetrachloride (0.5 mL, manufactured by Wako Pure Chemical Industries, Ltd.), and THF (8 mL) were added thereto. After cooling to 5°C or lower again, pyridine (1 mL) and THF (1 mL) were further added thereto. Thereafter, the temperature was raised to room temperature, and stirring was performed at room temperature for 18 hours and at 60°C for 14 hours. After completion of a reaction, the reaction solution was poured into a beaker containing water (50 mL), and toluene (30 mL) was added thereto. The reaction solution was separated, and the organic layer was washed twice with a 10% aqueous sodium carbonate solution (50 mL). The organic layer was dried with magnesium sulfate, and concentrated. The crude was purified with a silica gel column (toluene) to obtain a compound represented by the following formula (A-63) as a black green powder (yield: 0.51 g, yield rate: 77%).

$^1$H-NMR (400 MHz, $CDCl_3$): δ (ppm) = 3.81 (12H), 3.95 (3H), 6.84 (8H), 6.90 (4H), 7.07 (8H), 7.34 (4H), 7.68 (1H), 7.82 (1H).

[Chem. 37]

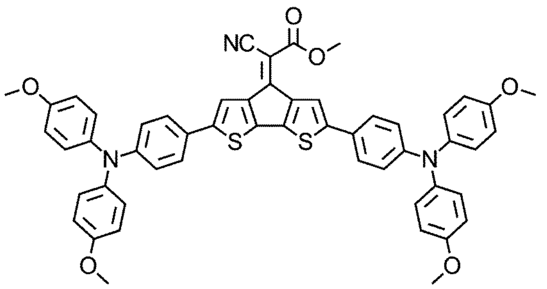

(A-63)

[Synthesis Example 9] Synthesis of Compound (A-66)

The compound represented by the formula (10) (0.88 g), 4-(diphenylamino)phenylboronic acid (1.73 g, manufactured by Sigma-Aldrich Co. LLC), tetrakis(triphenylphosphine)palladium (0) (0.12 g, manufactured by Kanto Chemical Co., Inc.), potassium carbonate (0.90 g, manufactured by Kanto Chemical Co., Inc.), toluene (13 mL), ethanol (4.5 mL), and water (4.5 mL) were added to a reaction vessel, and deaeration was performed under reduced pressure. The mixture was heated to reflux for 13 hours in an argon atmosphere. After completion of a reaction, the reaction solution was added to a beaker containing ethanol (50 mL). The mixture solution was filtered, and the residue was washed twice with water (50 mL), and then washed with ethanol (20 mL). The crude product was purified with a silica gel column (toluene and then chloroform). Thereafter, crystals were dispersed and washed with toluene to obtain a compound represented by the following formula (28) as a black blue powder (yield: 1.14 g, yield rate: 67%).

[Chem. 38]

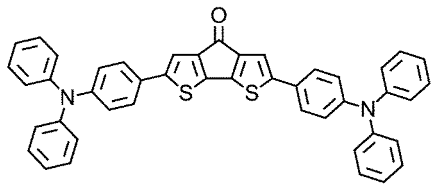

(28)

The compound represented by the formula (28) (0.60 g), malononitrile (0.23 g, manufactured by TCI), and THF (50 mL) were added to a reaction vessel and stirred in an argon atmosphere. A solution of acetic acid-pyridine (1:1) (5.6 mL) was added thereto. The mixture was stirred under heating and refluxing for 32 hours. After completion of a reaction, the reaction solution was added to a beaker containing water (300 mL). The mixture solution was filtered, and the residue was washed with water (20 mL) and methanol (20 mL). The crude product was dispersed and washed with acetone and recrystallized (chloroform) to obtain a compound represented by the following formula (A-66) as a black powder (yield: 0.44 g, yield rate: 69%).

$^1$H-NMR (400 MHz, THF-ds): δ (ppm) = 7.01 (8H), 7.06 (8H), 7.23 (8H), 7.45 (4H), 7.49 (2H).

[Chem. 39]

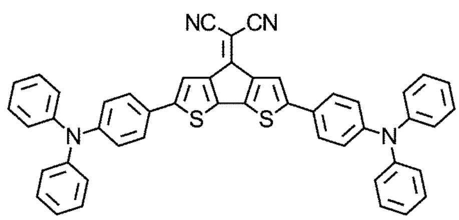

(A-66)

[Synthesis Example 10] Synthesis of Compound (A-98)

The compound represented by the formula (10) (0.88 g), a compound represented by the following formula (29) (2.55 g), potassium carbonate (0.90 g, manufactured by Kanto Chemical Co., Inc.), tetrakistriphenylphosphine palladium (0.12 g, manufactured by Kanto Chemical Co., Inc.), toluene (13 mL), ethanol (4.5 mL), and water (4.5 mL) were added to a reaction vessel, and heated to reflux for 6 hours. After completion of a reaction, the reaction solution

was added to a beaker containing water (150 mL), and toluene (20 mL) was further added, followed by liquid separation. The organic layer was dried with magnesium sulfate, and concentrated. The crude product was purified with a silica gel column (toluene) to obtain a compound represented by the following formula (30) as a black blue powder (yield: 1.81 g, yield rate: 84%).

[Chem. 40]

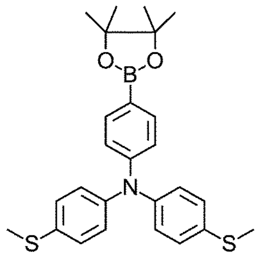

(29)

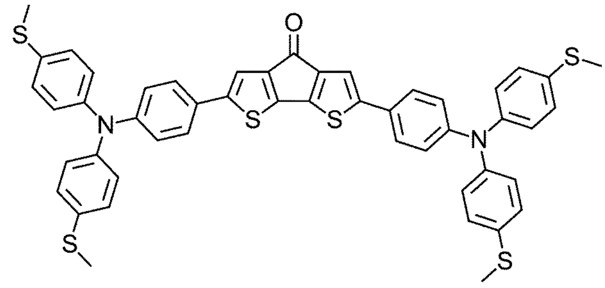

(30)

The compound represented by the formula (30) (0.60 g), malononitrile (0.22 g, manufactured by TCI), and THF (29 mL) were added to a reaction vessel and stirred in an argon atmosphere. A mixed solution of acetic acid and pyridine (volume ratio = 1:1) (5.0 mL) was added thereto, and the mixture was heated to reflux for 21 hours. After completion of a reaction, the reaction solution was added to a beaker containing water (150 mL). The mixture solution was filtered, and the solid was washed with water (20 mL) and methanol (20 mL). The crude product was purified with a silica gel column (toluene), and then recrystallized (chloroform:acetone) to obtain a compound represented by the following formula (A-98) as a black solid (yield: 0.54 g, yield rate: 84%).

$^1$H-NMR (400 MHz, $CDCl_3$): $\delta$ (ppm) = 2.48 (2H), 7.02 to 7.06 (12H), 7.18 to 7.21 (8H), 7.38 (4H), 7.45 (2H).

[Chem. 41]

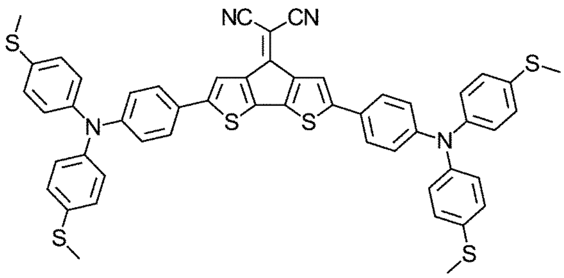

(A−98)

[Synthesis Example 11] Synthesis of Compound (A-114)

A compound represented by the following formula (31) (0.32 g), [4-[bis(4-methoxyphenyl)amino]phenyl]boronic acid (0.49 g, manufactured by TCI), tetrakis(triphenylphosphine)palladium (0) (0.03 g, manufactured by Kanto Chemical Co., Inc.), potassium carbonate (0.22 g, manufactured by Kanto Chemical Co., Inc.), toluene (4 mL), ethanol (1.3 mL), and water (1.3 mL) were added to a reaction vessel, and deaeration was performed under reduced pressure. The mixture was stirred under heating and refluxing for 6 hours in an argon atmosphere. After completion of a reaction, the reaction solution was added to water (100 mL). After 20 mL of toluene was added thereto, liquid separation was performed. The organic layer was dried with magnesium sulfate, and concentrated. The crude product was purified with a silica gel column (toluene) to obtain a compound represented by the following formula (32) as a black green powder (yield: 0.40 g, yield rate: 69%).

[Chem. 42]

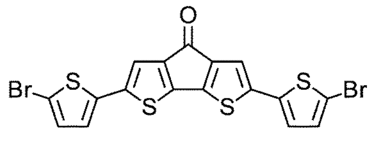

(31)

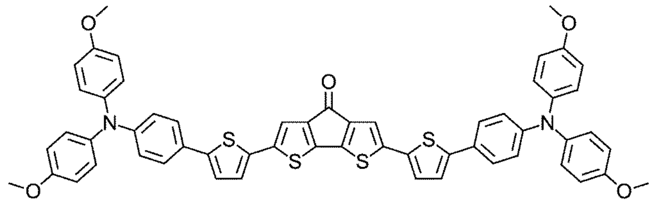

(32)

The compound represented by the formula (32) (0.35 g), diethyl malonate (0.27 mL, manufactured by TCI), and THF (11 mL) were added to a reaction vessel and stirred in an argon atmosphere. Pyridine (0.48 mL, manufactured by Nacalai Tesque, Inc.) was added thereto, and then titanium tetrachloride (0.24 mL, manufactured by Wako Pure Chemical Industries, Ltd.) was added thereto, followed by stirring at room temperature for 9 hours. After completion of a reaction, the reaction solution was added to a beaker containing water (150 mL). The mixture solution was filtered, and the residue was washed with water (30 mL) and methanol (20 mL), and dried. The crude product was purified with a silica gel column (toluene/ethyl acetate = 50/1 (volume ratio)) to obtain a compound represented by the following formula (A-114) as a black green powder (yield: 0.32 g, yield rate: 82%).

$^{1}$H-NMR (400 MHz, THF-ds): δ (ppm) = 1.35 (6H), 3.73 (12H), 4.39 (4H), 6.81 to 6.88 (12H), 7.02 (8H), 7.12 (2H), 7.15 (2H), 7.39 to 7.42 (6H).

[Chem. 43]

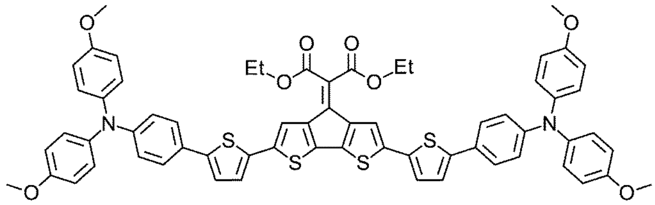

(A−114)

[Synthesis Example 12] Synthesis of Compound (A-115)

The compound represented by the formula (31) (0.32 g), a compound represented by the following formula (33) (1.15 g), tetrakis(triphenylphosphine)palladium (0) (0.03 g, manufactured by Kanto Chemical Co., Inc.), potassium carbonate (0.22 g, manufactured by Kanto Chemical Co., Inc.), toluene (4 mL), ethanol (1.3 mL), and water (1.3 mL) were added to a reaction vessel, and deaeration was performed under reduced pressure. The mixture was stirred under heating and refluxing for 6 hours in an argon atmosphere. After completion of a reaction, the reaction solution was added to water (100 mL). After toluene (20 mL) was added thereto, liquid separation was performed. The organic layer was dried with magnesium sulfate, and concentrated. The crude product was purified with a silica gel column (toluene/ethyl acetate = 40/1 (volume ratio)) to obtain a compound represented by the following formula (34) as a black green powder (yield: 0.64 g, yield rate: 61%).

[Chem. 44]

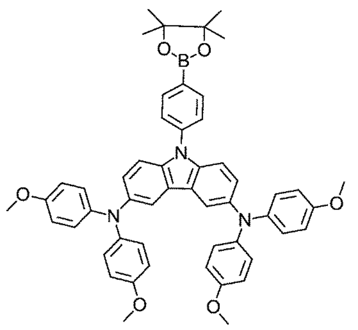

(33)

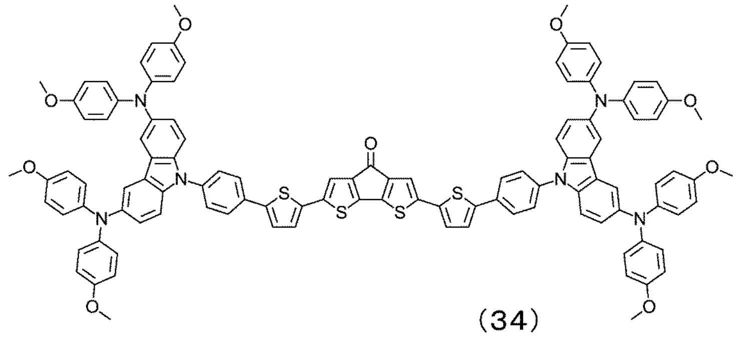

(34)

The compound represented by the formula (34) (0.32 g), malononitrile (0.05 g, manufactured by TCI), and THF (8 mL) were added to a reaction vessel and stirred in an argon atmosphere. A mixed solution of acetic acid and pyridine (volume ratio = 1:1) (1.4 mL) was added thereto, and the mixture was heated to reflux for 20 hours. After completion of a reaction, the reaction solution was poured into a beaker containing water (150 mL). The mixture solution was filtered, and the residue was washed with water (20 mL) and methanol (20 mL), and dried. The crude product was purified with a silica gel column (toluene/ethyl acetate = 40/1 (volume ratio)) to obtain a compound represented by the following formula (A-115) as a black green powder (yield: 0.26 g, yield rate: 81%).

$^1$H-NMR (400 MHz, THF-ds): $\delta$ (ppm) = 3.67 (24H), 6.69 to 6.71 (16H), 6.88 to 6.90 (16H), 7.07 (4H), 7.28 to 7.31 (6H), 7.47 (4H), 7.61 (4H), 7.64 (4H), 7.89 (4H).

[Chem. 45]

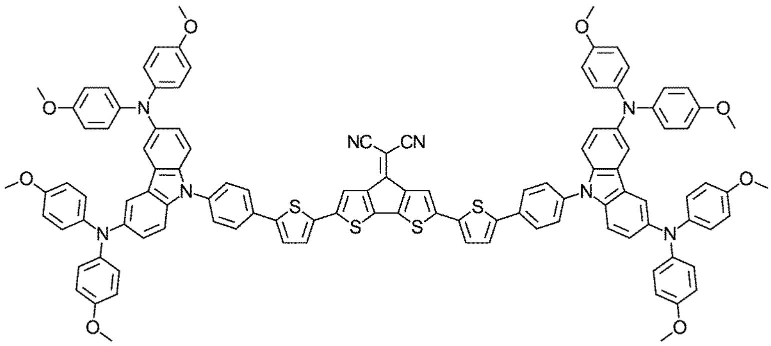

(A−115)

[Example 1] Production of Photoelectric Conversion Element and Evaluation of Photoelectric Conversion Characteristic

An etched glass substrate coated with a fluorine-doped tin oxide (FTO) thin film (conductive support 1, manufactured by Solaronix) was ultrasonically cleaned with acetone and then treated with UV ozone.

A titanium oxide (TiOz) solution for a hole blocking layer (Ti-Nanoxide BL/SC, manufactured by Solaronix) was applied onto the substrate by a spin coating method. After coating, firing was performed at 500°C for 1 hour by using an electric furnace to obtain a TiOz thin film (hole blocking layer 2) having a film thickness of 50 nm to 70 nm. Subsequently, a titanium oxide (TiOz) dispersion for an electron transport layer prepared at a ratio of titanium oxide (TiOz) paste (PST-18NR, manufactured by JGC Catalysts and Chemicals Ltd.):ethanol = 1:4 (weight ratio) was applied onto the substrate by a spin coating method. After coating, firing was performed at 450°C for 30 minutes by using an electric furnace to obtain a mesoporous titanium oxide (TiOz) thin film (electron transport layer 3) having a film thickness of 330 nm.

Next, $PbI_2$/MAI (1:1)-DMF complex (manufactured by Tokyo Chemical Industry Co., Ltd.) was dissolved in dimethyl sulfoxide to prepare a 1.5M solution. At room temperature, the prepared dimethyl sulfoxide solution was spin-coated on the mesoporous titanium oxide (TiOz) thin film, and 0.3 mL of chlorobenzene was dropped during the spin-coating to form a film, and the film was dried by heating at 110°C for 1 hour by using a hot plate, to form a $CH_3NH_3PbI_3$ layer (photoelectric conversion layer 4) having a thickness of about 500 nm.

Next, a chlorobenzene solution containing 75 mM of 4-tert-butylpyridine, which is a basic compound as an additive, and 12.5 mM of bis(trifluoromethanesulfonyl)imide lithium (LiTFSI, 0.5 equivalents), which is a dopant as an additive, was prepared as a doping solution. A 50 mM chlorobenzene solution was prepared by using the prepared

doping solution and the compound (A-1) obtained in Synthesis Example 1 as a hole transport material for a photoelectric conversion element, and used as a hole transport layer solution. The hole transport layer solution was spin-coated on the $CH_3NH_3PbI_3$ layer (photoelectric conversion layer 4) at room temperature, and dried by heating at 70°C for 30 minutes by using a hot plate to form a hole transport layer 5 having a thickness of about 300 nm.

A gold electrode (counter electrode 6) was provided on the hole transport layer 5 by forming a gold film having a thickness of 80 nm to 100 nm by a vacuum deposition method at a degree of vacuum of about $1 \times 10^{-4}$ Pa, thereby producing a photoelectric conversion element.

Light generated by a pseudo-sunlight irradiation device (OTENTO-SUN III type, manufactured by Bunkoukeiki Co., Ltd.) was emitted from a transparent electrode side of the photoelectric conversion element, and a current-voltage characteristic was measured by adjusting an intensity of light to 100 mW/cm$^2$ by using a source meter (Model 2400 General-Purpose Source Meter, manufactured by KEITHLEY). An initial photoelectric conversion efficiency was obtained based on the measured current-voltage characteristic value. The photoelectric conversion element was stored in a desiccator containing silica gel for 168 hours from the measurement of the initial photoelectric conversion efficiency, and a photoelectric conversion efficiency after storage (photoelectric conversion efficiency over time) was evaluated. As an evaluation of durability, a retention rate (%) of the photoelectric conversion efficiency after storage for 168 hours from the measurement of the initial photoelectric conversion efficiency was calculated according to the following equation (a-1). The calculated retention rate (%) is shown in Table 2.

[Equation 1]

$$\text{RETENTION RATE (\%)} = \frac{\text{PHOTOELECTRIC CONVERSION EFFICIENCY OVER TIME}}{\text{INITIAL PHOTOELECTRIC CONVERSION EFFICIENCY}} \times 100 \qquad (a-1)$$

[Example 2]

A 50 mM chlorobenzene solution was prepared by using the compound (A-11) obtained in Synthesis Example 2 as a hole transport material for a photoelectric conversion element, and used as a hole transport layer solution. A photoelectric conversion element was produced in the same manner as in Example 1 except that the compound (A-11) was used and that bis(trifluoromethanesulfonyl)imide lithium (LiTFSI), which is a dopant as an additive, and 4-tert-butylpyridine, which is a basic compound as an additive, were not used (0 mM), and a current-voltage characteristic was measured to obtain an initial photoelectric conversion efficiency. A photoelectric conversion efficiency (photoelectric conversion efficiency over time) of the photoelectric conversion element in Example 2 was obtained after storage in a desiccator containing silica gel for 168 hours from the measurement of the initial photoelectric conversion efficiency. Table 1 shows a ratio (hereinafter, referred to as a ratio with Comparative Example 1) of the photoelectric conversion efficiency after storage for 168 hours in Example 2 to a photoelectric conversion efficiency after storage for 168 hours in Comparative Example 1 to be described later (storage under the same conditions as in Example 2), and Table 2 shows a retention rate (%) of the photoelectric conversion efficiency after storage for 168 hours from the measurement of the initial conversion efficiency, as an evaluation of durability of the photoelectric conversion element in Example 2. Here, the ratio with Comparative Example 1 shown in Table 1 is a value obtained by dividing a photoelectric conversion efficiency (photoelectric conversion efficiency over time) after storage for 168 hours in each of Examples by the photoelectric conversion efficiency (photoelectric conversion efficiency over time) after storage for 168 hours in Comparative Example 1.

[Example 3]

A photoelectric conversion element was produced in the same manner as in Example 1 except that the compound (A-11) was used, and a current-voltage characteristic was measured to obtain an initial photoelectric conversion efficiency and a photoelectric conversion efficiency after storage for 168 hours. Table 1 shows a ratio of the photoelectric conversion efficiency after storage for 168 hours from the measurement of the initial photoelectric conversion efficiency to that in Comparative Example 1, and Table 2 shows, as an evaluation of durability, a retention rate (%) of the photoelectric conversion efficiency after storage for 168 hours from the measurement of the initial conversion efficiency.

[Example 4]

A photoelectric conversion element was produced in the same manner as in Example 2 except that the compound (A-56) was used, and a current-voltage characteristic was measured to obtain an initial photoelectric conversion efficiency and a photoelectric conversion efficiency after storage for 168 hours. Table 1 shows a ratio of the photoelectric conversion efficiency after storage for 168 hours from the measurement of the initial photoelectric conversion efficiency to that in

Comparative Example 1, and Table 2 shows, as an evaluation of durability, a retention rate (%) of the photoelectric conversion efficiency after storage for 168 hours from the measurement of the initial conversion efficiency.

[Example 5]

A photoelectric conversion element was produced in the same manner as in Example 1 except that the compound (A-56) was used, and a current-voltage characteristic was measured to obtain an initial photoelectric conversion efficiency and a photoelectric conversion efficiency after storage for 168 hours. Table 1 shows a ratio of the photoelectric conversion efficiency after storage for 168 hours from the measurement of the initial photoelectric conversion efficiency to that in Comparative Example 1, and Table 2 shows, as an evaluation of durability, a retention rate (%) of the photoelectric conversion efficiency after storage for 168 hours from the measurement of the initial conversion efficiency.

[Example 6]

A photoelectric conversion element was produced in the same manner as in Example 2 except that the compound (A-63) was used, and a current-voltage characteristic was measured to obtain an initial photoelectric conversion efficiency and a photoelectric conversion efficiency after storage for 168 hours. Table 1 shows a ratio of the photoelectric conversion efficiency after storage for 168 hours from the measurement of the initial conversion efficiency to that in Comparative Example 1, and Table 2 shows, as an evaluation of durability, a retention rate (%) of the photoelectric conversion efficiency after storage for 168 hours from the measurement of the initial conversion efficiency.

[Example 7]

A photoelectric conversion element was produced in the same manner as in Example 1 except that the compound (A-63) was used, and a current-voltage characteristic was measured to obtain an initial photoelectric conversion efficiency and a photoelectric conversion efficiency after storage for 168 hours. Table 1 shows a ratio of the photoelectric conversion efficiency after storage for 168 hours from the measurement of the initial conversion efficiency to that in Comparative Example 1.

[Comparative Example 1]

A photoelectric conversion element was produced in the same manner as in Example 1 except that Spiro-OMeTAD (manufactured by Sigma-Aldrich), which is a standard hole transport material represented by the following formula (B-1), was used, and a current-voltage characteristic was measured to obtain an initial photoelectric conversion efficiency and the photoelectric conversion efficiency after storage for 168 hours. The initial photoelectric conversion efficiency was 6.03%. Table 2 shows, as an evaluation of durability, a retention rate (%) of the photoelectric conversion efficiency after storage for 168 hours from the measurement of the initial photoelectric conversion efficiency.

[Chem. 46]

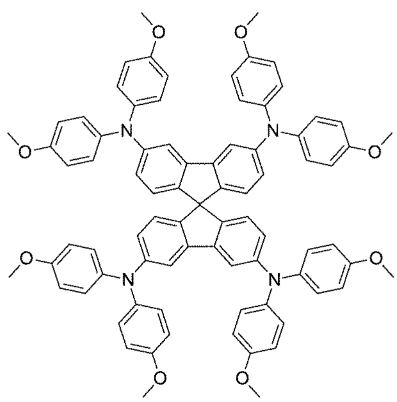

（B－1）

[Table 1]

| | Compound | Addition amount of dopant (LiTFSI) [equivalent] | Ratio of photoelectric conversion efficiency to that in Comparative Example 1 (after 168 hours) |
|---|---|---|---|
| Example 2 | A-11 | 0 | 1.04 |
| Example 3 | A-11 | 0.5 | 1.15 |
| Example 4 | A-56 | 0 | 1.55 |
| Example 5 | A-56 | 0.5 | 1.97 |
| Example 6 | A-63 | 0 | 0.97 |
| Example 7 | A-63 | 0.5 | 0.92 |
| Comparative Example 1 | B-1 | 0.5 | 1 |

[Table 2]

| | Compound | Addition amount of dopant (LiTFSI) [equivalent] | Retention rate [%] |
|---|---|---|---|
| Example 1 | A-1 | 0.5 | 80% |
| Example 2 | A-11 | 0 | 103% |
| Example 3 | A-11 | 0.5 | 107% |
| Example 4 | A-56 | 0 | 117% |
| Example 5 | A-56 | 0.5 | 83% |
| Example 6 | A-63 | 0 | 106% |
| Comparative Example 1 | B-1 | 0.5 | 72% |

From results shown in Table 1, it was found that the compound (A-11) and the compound (A-56) exhibited sufficient photoelectric conversion efficiencies higher than that of the compound in the Comparative Example which is used as a standard. In addition, it was found that the compound (A-63) exhibited a high photoelectric conversion efficiency equivalent to that of the compound in the Comparative Example which is used as a standard. Furthermore, it was found that the compound (A-11), the compound (A-56), and the compound (A-63) can be used in the hole transport layer of the photoelectric conversion element even if the dopant as an additive was reduced or not used. Accordingly, the photoelectric conversion element containing the compound (A-11), the compound (A-56), or the compound (A-63) can be produced at a reduced cost by reducing the amount of doping, and can be produced with a simpler process and at a lower cost without requiring a doping operation.

From results shown in Table 2, as for durability over time of the photoelectric conversion element using the hole transport layer containing the compound (A-1), the compound (A-11), the compound (A-56), or the compound (A-63), it was found that the photoelectric conversion efficiency was at a high level even after 168 hours, and the retention rate thereof was superior to that of the compound in the Comparative Example.

[Example 8] Production of Photoelectric Conversion Element and Evaluation of Current-Voltage Characteristic

Glass with a FLAT ITO film (conductive support 7, manufactured by Geomatec Co., Ltd.) was ultrasonically cleaned with isopropyl alcohol and then treated with UV ozone.

A tin oxide dispersion (electron transport layer coating liquid) in which Tin (IV) oxide, 15% in $H_2O$ colloidal dispersion (manufactured by Alfa Aesar) and purified water were mixed at a volume ratio of 1:9 was applied onto the substrate by spin coating. Thereafter, heating was performed at 150°C for 30 minutes with a hot plate to form an electron transport layer 8 having a film thickness of about 20 nm.

In a glove box under a nitrogen stream, formamidine hydroiodide (1 M, manufactured by Tokyo Chemical Industry Co., Ltd.), lead (II) iodide (1.1 M, manufactured by Tokyo Chemical Industry Co., Ltd.), methylamine hydrobromide (0.2 M, manufactured by Tokyo Chemical Industry Co., Ltd.), and lead (II) bromide (0.2 M, manufactured by Tokyo Chemical Industry Co., Ltd.) were dissolved in a mixed solvent containing dimethyl formamide and dimethyl sulfoxide at a volume ratio of 4:1. A dimethyl sulfoxide solution of cesium iodide (1.5 M, manufactured by Tokyo Chemical Industry

Co., Ltd.) was added thereto such that the amount of cesium charged was 5% in terms of composition ratio, to prepare a perovskite precursor solution.

In a glove box under a nitrogen atmosphere, the prepared perovskite precursor solution was dropped onto the tin oxide thin film and spin-coated, and 0.3 mL of chlorobenzene was dropped during the spin coating to apply a perovskite precursor. Thereafter, heating was performed at 100°C for 1 hour with a hot plate to form a $Cs(MAFA)Pb(IBr)_3$ layer (photoelectric conversion layer 9) having a film thickness of about 500 nm.

In a glove box under a nitrogen stream, a chlorobenzene solution of a dopant containing 150 mM of 4-tert-butylpyridine and 25 mM of bis(trifluoromethanesulfonyl)imide lithium (LiTFSI, 0.5 equivalents) was prepared. The compound (A-63) obtained in Synthesis Example 11 as a hole transport material was dissolved in the chlorobenzene solution at room temperature at 50 mM to prepare a coating solution for a hole transport layer.

In a glove box under a nitrogen atmosphere, the coating solution for a hole transport layer was spin-coated on the $Cs(MAFA)Pb(IBr)_3$ layer (photoelectric conversion layer 9) to form a hole transport layer 10 having a film thickness of about 200 nm.

A gold electrode (counter electrode 11) was formed on the hole transport layer 10 by forming a gold film having a thickness of about 80 nm by a vacuum deposition method at a degree of vacuum of about $1 \times 10^{-4}$ Pa, thereby producing a photoelectric conversion element.

Light having an intensity of 100 $mW/cm^2$ generated by a pseudo-sunlight irradiation device (OTENTO-SUN III type, manufactured by Bunkoukeiki Co., Ltd.) was emitted from a conductive support side of the photoelectric conversion element, and a current-voltage characteristic was measured by using a source meter (Model 2400 General-Purpose Source Meter, manufactured by KEITHLEY) to obtain an initial photoelectric conversion efficiency. The photoelectric conversion element was stored in a desiccator containing silica gel for 30 days from the measurement of the initial photoelectric conversion efficiency, and a current-voltage characteristic was measured again to obtain a photoelectric conversion efficiency after 30 days (after 720 hours). Table 3 shows a ratio (hereinafter, referred to as a ratio with Comparative Example 2) of the photoelectric conversion efficiency after storage for 720 hours as photoelectric conversion over time from the measurement of the initial photoelectric conversion efficiency of the photoelectric conversion element in Example 8 to a photoelectric conversion efficiency of a photoelectric conversion element in Comparative Example 2 to be described later after storage in the same manner as the photoelectric conversion element in Example 8, and Table 4 shows, as an evaluation of durability, a retention rate (%) of the photoelectric conversion efficiency after storage for 720 hours from the measurement of the initial photoelectric conversion efficiency.

[Example 9]

A 50 mM chlorobenzene solution was prepared by using the compound (A-63) obtained in Synthesis Example 11 as a hole transport material for a photoelectric conversion element, and used as a hole transport layer solution. A photoelectric conversion element was produced in the same manner as in Example 8 except that the hole transport layer solution was used in which bis(trifluoromethanesulfonyl)imide lithium (LiTFSI), which is a dopant as an additive, and 4-tert-butylpyridine, which is a basic compound as an additive, were not used (0 mM), and a current-voltage characteristic was measured to obtain an initial photoelectric conversion efficiency. The photoelectric conversion element was stored in a desiccator containing silica gel for 30 days from the measurement of the initial photoelectric conversion efficiency, and a current-voltage characteristic was measured again to obtain a photoelectric conversion efficiency after 30 days (after 720 hours). Table 3 shows a ratio of the photoelectric conversion efficiency after storage for 720 hours as photoelectric conversion over time from the measurement of the initial photoelectric conversion efficiency to that in Comparative Example 2, and Table 4 shows, as an evaluation of durability, a retention rate (%) of the photoelectric conversion efficiency after storage for 720 hours from the measurement of the initial photoelectric conversion efficiency.

[Example 10]

A photoelectric conversion element was produced in the same manner as in Example 8 except that the compound (A-114) was used, and a current-voltage characteristic was measured to obtain an initial photoelectric conversion efficiency and a photoelectric conversion efficiency after 30 days (after 720 hours). Table 3 shows a ratio of the photoelectric conversion efficiency after storage for 720 hours from the measurement of the initial photoelectric conversion efficiency to that in Comparative Example 2, and Table 4 shows, as an evaluation of durability, a retention rate (%) of the photoelectric conversion efficiency after storage for 720 hours from the measurement of the initial conversion efficiency.

[Example 11]

A photoelectric conversion element was produced in the same manner as in Example 9 except that the compound (A-114) was used, and a current-voltage characteristic was measured to obtain an initial photoelectric conversion efficiency and a photoelectric conversion efficiency after 30 days (after 720 hours). Table 3 shows a ratio of the photoelectric conversion efficiency after storage for 720 hours from the measurement of the initial conversion efficiency to that in Comparative Example 1, and Table 4 shows, as an evaluation of durability, a retention rate (%) of the photoelectric conversion efficiency after storage for 720 hours from the measurement of the initial conversion efficiency.

[Example 12]

A photoelectric conversion element was produced in the same manner as in Example 8 except that the compound (A-115) was used, and a current-voltage characteristic was measured to obtain an initial photoelectric conversion efficiency and a photoelectric conversion efficiency after 30 days (after 720 hours). Table 3 shows a ratio of the photoelectric conversion efficiency after storage for 720 hours from the measurement of the initial photoelectric conversion efficiency to that in Comparative Example 2, and Table 4 shows, as an evaluation of durability, a retention rate (%) of the photoelectric conversion efficiency after storage for 720 hours from the measurement of the initial conversion efficiency.

[Example 13]

A photoelectric conversion element was produced in the same manner as in Example 9 except that the compound (A-115) was used, and a current-voltage characteristic was measured to obtain an initial photoelectric conversion efficiency and a photoelectric conversion efficiency after 30 days (after 720 hours). Table 3 shows a ratio of the photoelectric conversion efficiency after storage for 720 hours from the measurement of the initial conversion efficiency to that in Comparative Example 1, and Table 4 shows, as an evaluation of durability, a retention rate (%) of the photoelectric conversion efficiency after storage for 720 hours from the measurement of the initial conversion efficiency.

[Comparative Example 2]

A photoelectric conversion element was produced in the same manner as in Example 8 except that Spiro-OMeTAD (manufactured by Sigma-Aldrich), which is a standard hole transport material represented by the formula (B-1) shown in Comparative Example 1, was used, and a current-voltage characteristic was measured to obtain an initial photoelectric conversion efficiency and a photoelectric conversion efficiency after 30 days (after 720 hours). The initial photoelectric conversion efficiency was 13.2%. Table 4 shows a retention rate (%) of the photoelectric conversion efficiency after storage for 720 hours from the measurement of the initial conversion efficiency.

[Table 3]

| | Compound | Addition amount of dopant (LiTFSI) [equivalent] | Ratio of photoelectric conversion efficiency to that in Comparative Example 2 (after 720 hours) |
|---|---|---|---|
| Example 8 | A-63 | 0.5 | 1.58 |
| Example 9 | A-63 | 0 | 1.46 |
| Example 10 | A-114 | 0.5 | 0.81 |
| Example 11 | A-114 | 0 | 1.19 |
| Example 12 | A-115 | 0.5 | 1.85 |
| Example 13 | A-115 | 0 | 1.19 |
| Comparative Example 2 | B-1 | 0.5 | 1 |

[Table 4]

| | Compound | Addition amount of dopant (LiTFSI) [equivalent] | Retention rate [%] |
|---|---|---|---|
| Example 8 | A-63 | 0.5 | 105% |
| Example 9 | A-63 | 0 | 95% |
| Example 10 | A-114 | 0.5 | 95% |
| Example 11 | A-114 | 0 | 165% |

(continued)

| | Compound | Addition amount of dopant (LiTFSI) [equivalent] | Retention rate [%] |
|---|---|---|---|
| Example 12 | A-115 | 0.5 | 110% |
| Example 13 | A-115 | 0 | 84% |
| Comparative Example 2 | B-1 | 0.5 | 70% |

From results shown in Table 3, it was found that the compound (A-63), the compound (A-114), and the compound (A-115) exhibited sufficient photoelectric conversion efficiencies higher than that of a comparative compound which is used as a standard. Furthermore, it was found that the compound (A-63), the compound (A-114), and the compound (A-115) can be used in the hole transport layer of the photoelectric conversion element even if the dopant as an additive was reduced or not used. Accordingly, the photoelectric conversion element containing the compound (A-63), the compound (A-114), or the compound (A-115) can be produced at a reduced cost by reducing the amount of doping, and can be produced with a simpler process and at a lower cost without requiring a doping operation.

From results shown in Table 4, as for durability over time of the photoelectric conversion element using the hole transport layer containing the compound (A-63), the compound (A-114), or the compound (A-115), it was found that the photoelectric conversion efficiency was at a high level even after 720 hours, and the retention rate thereof was superior to that of the compound in the Comparative Example.

Although the present invention has been described in detail with reference to specific aspects, it is apparent to those skilled in the art that it is possible to add various alterations and modifications without departing from the spirit and the scope of the present invention.

The present application is based on Japanese Patent Application No. 2020-129304 filed on July 30, 2020, the entirety of which is incorporated by reference. In addition, all references cited here are entirely incorporated.

## INDUSTRIAL APPLICABILITY

Use of the hole transport layer for a photoelectric conversion element according to the present invention is useful for a photoelectric conversion element and a perovskite solar cell with high efficiency and high durability capable of efficiently extracting a current, and it is possible to provide clean energy as a solar cell which can efficiently convert solar energy into electric energy.

## REFERENCE SIGNS LIST

1: conductive support
2: hole blocking layer
3: electron transport layer
4: photoelectric conversion layer
5: hole transport layer
6: counter electrode
7: conductive support
8: electron transport layer
9: photoelectric conversion layer
10: hole transport layer
11: counter electrode

## Claims

**1.** A compound represented by the following general formula (1), wherein $X^1$ and $X^2$ in the general formula (1) are each independently represented by the following general formula (2):

[Chem. 1]

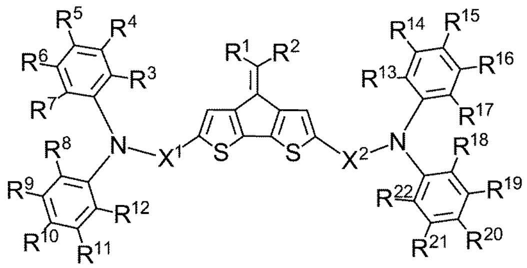

(1)

(wherein, $R^1$ and $R^2$ each independently represent

a nitrile group,
a linear or branched perfluoroalkyl group having 1 to 4 carbon atoms,
a linear or branched acyl group having 1 to 18 carbon atoms which may have a substituent,
a linear or branched alkoxycarbonyl group having 1 to 18 carbon atoms which may have a substituent,
a thio group having 1 to 18 carbon atoms which may have a substituent,
an amino group having 1 to 20 carbon atoms which may have a substituent, or
a linear or branched alkylsulfonyl group having 1 to 18 carbon atoms which may have a substituent;

$R^3$ to $R^{22}$ each independently represent

a hydrogen atom, a halogen atom, a carboxy group, a trimethylsilyl group,
a linear or branched alkyl group having 1 to 18 carbon atoms which may have a substituent,
a linear or branched alkenyl group having 2 to 20 carbon atoms which may have a substituent,
a linear or branched alkynyl group having 2 to 20 carbon atoms which may have a substituent,
a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
an acyl group having 1 to 20 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms and having a substituent, or
a heterocyclic group having 5 to 36 ring atoms which may have a substituent;

$R^1$ and $R^2$ may be bonded to each other to form a ring;
$R^3$ to $R^7$, $R^8$ to $R^{12}$, $R^{13}$ to $R^{17}$, and $R^{18}$ to $R^{22}$ may be bonded to each other between adjacent groups to form a ring; and
$R^7$ and $R^8$, and $R^{17}$ and $R^{18}$ may be bonded to each other to form a ring, and
here, when $R^1$ and $R^2$ are a nitrile group, and $R^5$ is a methoxy group, $R^{10}$, $R^{15}$, and $R^{20}$ are at least one of the above groups other than a methoxy group), and

[Chem. 2]

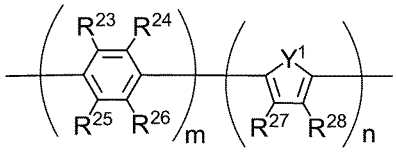

(2)

(wherein, $R^{23}$ to $R^{28}$ each independently represent

a hydrogen atom,
a linear or branched alkyl group having 1 to 20 carbon atoms which may have a substituent,
a linear or branched alkenyl group having 2 to 20 carbon atoms which may have a substituent,
a linear or branched alkynyl group having 2 to 20 carbon atoms which may have a substituent,

a cycloalkyl group having 3 to 10 carbon atoms which may have a substituent,
a linear or branched alkoxy group having 1 to 20 carbon atoms which may have a substituent,
a cycloalkoxy group having 3 to 10 carbon atoms which may have a substituent,
a thio group having 1 to 18 carbon atoms which may have a substituent,
an amino group having 1 to 20 carbon atoms which may have a substituent,
an aromatic hydrocarbon group having 6 to 36 carbon atoms which may have a substituent, or
a heterocyclic group having 5 to 36 ring atoms which may have a substituent;

$R^{23}$ and $R^{24}$, $R^{25}$ and $R^{26}$, and $R^{27}$ and $R^{28}$ may be bonded to each other to form a ring;
$Y^1$ represents an oxygen atom, a sulfur atom, or a selenium atom; and
m and n each represent an integer of 0 to 2, and
here, any one of m and n is 1 or 2).

**2.** The compound according to Claim 1, wherein in the general formula (1), $R^1$ and $R^2$ are each independently

a nitrile group,
a linear or branched acyl group having 1 to 18 carbon atoms which may have a substituent, or
a linear or branched alkoxycarbonyl group having 1 to 18 carbon atoms which may have a substituent.

**3.** The compound according to Claim 1 or 2, wherein m in the general formula (2) is 1.

**4.** A hole transport material for a photoelectric conversion element, the material comprising the compound described in any one of Claims 1 to 3.

**5.** A hole transport layer, comprising: a composition comprising the hole transport material for a photoelectric conversion element described in Claim 4, and a dopant as an additive in an amount of 0 equivalents to 0.5 equivalents with respect to 1 equivalent of the hole transport material for a photoelectric conversion element.

**6.** A photoelectric conversion element, comprising the hole transport layer described in Claim 5.

**7.** A solar cell, comprising the photoelectric conversion element described in Claim 6.

[FIG. 1]

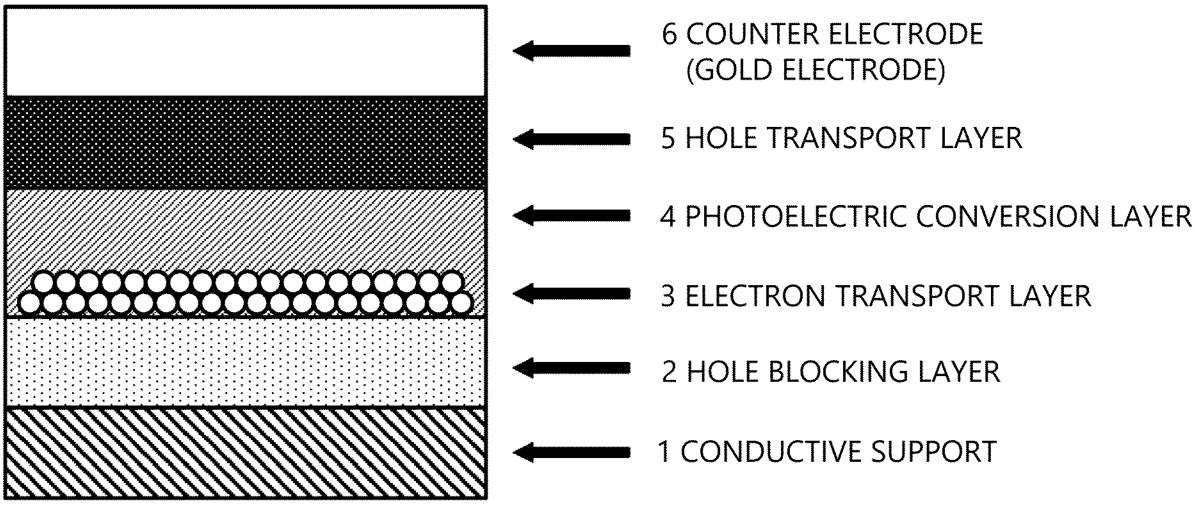
6 COUNTER ELECTRODE
(GOLD ELECTRODE)
5 HOLE TRANSPORT LAYER
4 PHOTOELECTRIC CONVERSION LAYER
3 ELECTRON TRANSPORT LAYER
2 HOLE BLOCKING LAYER
1 CONDUCTIVE SUPPORT

[FIG. 2]

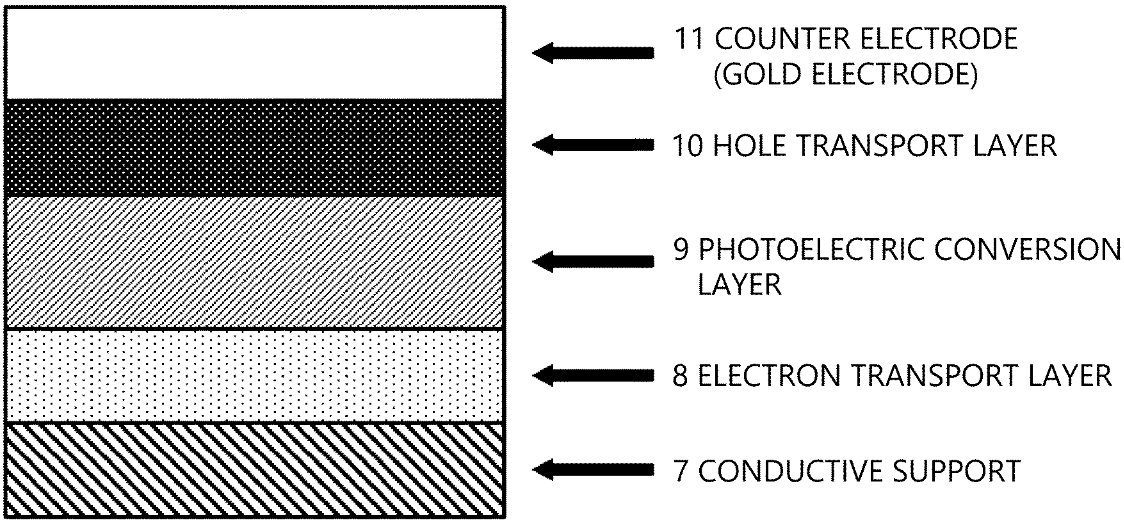
11 COUNTER ELECTRODE
(GOLD ELECTRODE)
10 HOLE TRANSPORT LAYER
9 PHOTOELECTRIC CONVERSION
LAYER
8 ELECTRON TRANSPORT LAYER
7 CONDUCTIVE SUPPORT

## INTERNATIONAL SEARCH REPORT

International application No.
PCT/JP2021/027782

A. CLASSIFICATION OF SUBJECT MATTER
Int.Cl. C07D495/04(2006.01)i, H01L51/46(2006.01)i
FI: C07D495/04101, H01L31/04168, H01L31/04154C, H01L31/04154B, C07D495/04CSP
According to International Patent Classification (IPC) or to both national classification and IPC

B. FIELDS SEARCHED
Minimum documentation searched (classification system followed by classification symbols)
Int.Cl. C07D495/04, H01L51/46

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | CN 109438469 A (QINGDAO UNIVERSITY OF SCIENCE & TECHNOLOGY) 08 March 2019 (2019-03-08), paragraph [0020] | 1-3<br>4-7 |
| X<br>Y | Journal of Materials Chemistry A, 19 December 2019, vol. 8, no. 3, pp. 1386-1393, fig. 1 | 1, 3-7<br>1-7 |
| Y | ACS Applied Energy Materials, 2019, vol. 2, no. 10, pp. 7070-7082, fig. 1 | 1-7 |
| Y | CN 106432265 A (CHANGCHUN INSTITUTE OF APPLIED CHEMISTRY, CHINESE ACADEMY OF SCIENCES) 22 February 2017 (2017-02-22), paragraph [0018] | 1-7 |
| Y | Energy Technology, 2019, vol. 7, no. 2, pp. 307-316, fig. 1 | 1-7 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 31 August 2021 | 14 September 2021 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2021/027782

| | | |
|---|---|---|
| CN 109438469 A | 08 March 2019 | (Family: none) |
| CN 106432265 A | 22 February 2017 | (Family: none) |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017104792 A
- JP 2020129304 A


### Non-patent literature cited in the description


- *Journal of the American Chemical Society,* 2009, vol. 131, 6050-6051
- *Science,* 2012, vol. 388, 643-647
- *Chem. Sci.,* 2019, vol. 10, 6748-6769
- *Adv. Funct. Mater.,* 2019, 1901296